# EUROPEAN PATENT APPLICATION

(11) **EP 3 587 563 A1**
(43) Date of publication of application: **01.01.2020**
(21) Application number: 18758462.8
(22) Date of filing: 23.02.2018
(51) Int. Cl.: C12N 5/079, C12N 5/0735, C12N 5/10, C12N 15/12, C12N 15/88

(54) **NERVE CELL PRODUCTION METHOD**

(30) Priority: 24.02.2017 US 201762463432 P
(71) Applicant: Tanabe, Koji, Palo Alto, CA 94303 (US); I Peace, Inc., Palo Alto, CA 94303 (US)
(72) Inventor: TANABE, Koji, Palo Alto California 94303 (US); KELLY, Brendan, Palo Alto California 94303 (US)
(74) Representative: MacLean, Martin Robert
(86) International application number: PCT/JP2018/006660
(87) International publication number: WO 2018/155620

(57) **Abstract**

The present invention provides a nerve cell production method that involves preparing stem cells and then introducing inducing-factor RNA into the stem cells and allowing differentiation into nerve cells. The present invention also provides a nerve cell production method that involves preparing cells, introducing reprogramming-factor RNA into the cells, introducing inducing-factor RNA into the cells into which the reprogramming-factor RNA was introduced, and allowing differentiation into nerve cells.

SEQUENCE LISTING
<110> I Peace, Inc.

<120> Method for manufacturing a specific somatic cell from an animal cell

<130> XT374AJP0005-US

<160> 10

<170> PatentIn version 3.5.1

<210> 1
<211> 1479
<212> DNA
<213> Artificial Sequence

<220>
<223> Inducing factor

<400> 1

atggactaca aggacgacga tgacaagttc gtcaaatctg agactctgga gttgaaggag 60
gaagaggagg tactgatgct gctgggctcg gcttccccgg cctcggcgac cctgaccccg 120
atgtcctcca gcgcggacga ggaggaggac gaggagctgc gccggccggg ctccgcgcgt 180
gggcagcgtg gagcggaagc cgggcagggg gtgcagggca gtccggcgtc gggtgccggg 240
ggttgccggc cagggcggct gctgggcctg atgcacgagt gcaagcgtcg cccgtcgcgc 300
tcacgggccg tctcccgagg tgccaagacg gcggagacgg tgcagcgcat caagaagacc 360
cgcaggctca aggccaacaa ccgcgagcgc aaccgcatgc acaacctaaa cgccgcgctg 420
gacgcgctgc gcgaggtgct gcccaccttc cccgaggatg ccaagctcac gaagatcgag 480
acgctgcgct tcgcccacaa ttacatctgg gcgctcaccg agactctgcg cctggcggac 540
cactgcgccg gcgccggtgg cctccagggg gcgctcttca cggaggcggt gctcctgagc 600
ccgggagctg cgctcggcgc cagcggggac agcccttctc caccttcctc ctggagctgc 660
accaacagcc cggcgtcatc ctccaactcc acgtccccat acagctgcac tttatcgccc 720
gctagccccg ggtcagacgt ggactactgg cagcccccac ctccggagaa gcatcgttat 780
gcgcctcacc tgcccctcgc cagggactgt atctctagag agggcagggg aagtcttcta 840
acatgcgggg acgtggagga aaatcccggc ccactcgaga tgaccgagta caagcccacg 900
gtgcgcctcg ccacccgcga cgacgtcccc agggccgtac gcaccctcgc cgccgcgttc 960
gccgactacc ccgccacgcg ccacaccgtc gatccggacc gccacatcga gcgggtcacc 1020
gagctgcaag aactcttcct cacgcgcgtc gggctcgaca tcggcaaggt gtgggtcgcg 1080
gacgacggcg ccgcggtggc ggtctggacc acgccggaga gcgtcgaagc gggggcggtg 1140
ttcgccgaga tcggcccgcg catggccgag ttgagcggtt cccggctggc cgcgcagcaa 1200
cagatggaag gcctcctggc gccgcaccgg cccaaggagc ccgcgtggtt cctggccacc 1260
gtcggcgtct cgcccgacca ccagggcaag ggtctgggca gcgccgtcgt gctccccgga 1320
gtggaggcgg ccgagcgcgc cggggtgccc gccttcctgg agacctccgc gccccgcaac 1380
ctccccttct acgagcggct cggcttcacc gtcaccgccg acgtcgaggt gcccgaagga 1440
ccgcgcacct ggtgcatgac ccgcaagccc ggtgcctga 1479

<210> 2
<211> 1473
<212> DNA
<213> Artificial Sequence

<220>
<223> Inducing factor

<400> 2

atggactaca aggacgacga tgacaagttc gtcaaatctg agactctgga gttgaaggag 60
gaagaggagg tactgatgct gctgggctcg gcttccccgg cctcggcgac cctgaccccg 120
atgtcctcca gcgcggacga ggaggaggac gaggagctgc gccggccggg ctccgcgcgt 180
gggcagcgtg gagcggaagc cgggcagggg gtgcagggca gtccggcgtc gggtgccggg 240
ggttgccggc cagggcggct gctgggcctg atgcacgagt gcaagcgtcg cccgtcgcgc 300

tcacgggccg tctcccgagg tgccaagacg gcggagacgg tgcagcgcat caagaagacc 360
cgcaggctca aggccaacaa ccgcgagcgc aaccgcatgc acaacctaaa cgccgcgctg 420
gacgcgctgc gcgaggtgct gcccaccttc cccgaggatg ccaagctcac gaagatcgag 480
acgctgcgct tcgcccacaa ttacatctgg gcgctcaccg agactctgcg cctggcggac 540
cactgcgccg gcgccggtgg cctccagggg gcgctcttca cggaggcggt gctcctgagc 600
ccgggagctg cgctcggcgc cagcggggac agcccttctc caccttcctc ctggagctgc 660
accaacagcc cggcgtcatc ctccaactcc acgtccccat acagctgcac tttatcgccc 720
gctagccccg ggtcagacgt ggactactgg cagcccccac ctccggagaa gcatcgttat 780
gcgcctcacc tgcccctcgc cagggactgt atcgagggca ggggaagtct tctaacatgc 840
ggggacgtgg aggaaaatcc cggcccactc gagatgaccg agtacaagcc cacggtgcgc 900
ctcgccaccc gcgacgacgt ccccagggcc gtacgcaccc tcgccgccgc gttcgccgac 960
taccccgcca cgcgccacac cgtcgatccg gaccgccaca tcgagcgggt caccgagctg 1020
caagaactct tcctcacgcg cgtcgggctc gacatcggca aggtgtgggt cgcggacgac 1080
ggcgccgcgg tggcggtctg gaccacgccg gagagcgtcg aagcgggggc ggtgttcgcc 1140
gagatcggcc cgcgcatggc cgagttgagc ggttcccggc tggccgcgca gcaacagatg 1200
gaaggcctcc tggcgccgca ccggcccaag gagcccgcgt ggttcctggc caccgtcggc 1260
gtctcgcccg accaccaggg caagggtctg ggcagcgccg tcgtgctccc cggagtggag 1320
gcggccgagc gcgccggggt gcccgccttc ctggagacct ccgcgccccg caacctcccc 1380
ttctacgagc ggctcggctt caccgtcacc gccgacgtcg aggtgcccga aggaccgcgc 1440
acctggtgca tgacccgcaa gcccggtgcc tga 1473

<210> 3
<211> 1488
<212> DNA
<213> Artificial Sequence

<220>
<223> Inducing factor

<400> 3

atggactaca aggacgacga tgacaagttc gtcaaatctg agactctgga gttgaaggag 60
gaagaggagg tactgatgct gctgggctcg gcttccccgg cctcggcgac cctgaccccg 120
atgtcctcca gcgcggacga ggaggaggac gaggagctgc gccggccggg ctccgcgcgt 180
gggcagcgtg gagcggaagc cgggcagggg gtgcagggca gtccggcgtc gggtgccggg 240
ggttgccggc cagggcggct gctgggcctg atgcacgagt gcaagcgtcg cccgtcgcgc 300
tcacgggccg tctcccgagg tgccaagacg gcggagacgg tgcagcgcat caagaagacc 360
cgcaggctca aggccaacaa ccgcgagcgc aaccgcatgc acaacctaaa cgccgcgctg 420
gacgcgctgc gcgaggtgct gcccaccttc cccgaggatg ccaagctcac gaagatcgag 480
acgctgcgct tcgcccacaa ttacatctgg gcgctcaccg agactctgcg cctggcggac 540
cactgcgccg gcgccggtgg cctccagggg gcgctcttca cggaggcggt gctcctgagc 600
ccgggagctg cgctcggcgc cagcggggac agcccttctc caccttcctc ctggagctgc 660
accaacagcc cggcgtcatc ctccaactcc acgtccccat acagctgcac tttatcgccc 720
gctagccccg ggtcagacgt ggactactgg cagcccccac ctccggagaa gcatcgttat 780
gcgcctcacc tgcccctcgc cagggactgt atctctagag gctccggcga gggcagggga 840
agtcttctaa catgcgggga cgtggaggaa aatcccggcc cactcgagat gaccgagtac 900
aagcccacgg tgcgcctcgc cacccgcgac gacgtcccca gggccgtacg caccctcgcc 960
gccgcgttcg ccgactaccc cgccacgcgc cacaccgtcg atccggaccg ccacatcgag 1020
cgggtcaccg agctgcaaga actcttcctc acgcgcgtcg ggctcgacat cggcaaggtg 1080
tgggtcgcgg acgacggcgc cgcggtggcg gtctggacca cgccggagag cgtcgaagcg 1140
ggggcggtgt tcgccgagat cggcccgcgc atggccgagt tgagcggttc ccggctggcc 1200
gcgcagcaac agatggaagg cctcctggcg ccgcaccggc ccaaggagcc cgcgtggttc 1260
ctggccaccg tcggcgtctc gcccgaccac cagggcaagg gtctgggcag cgccgtcgtg 1320
ctccccggag tggaggcggc cgagcgcgcc ggggtgcccg ccttcctgga gacctccgcg 1380
ccccgcaacc tccccttcta cgagcggctc ggcttcaccg tcaccgccga cgtcgaggtg 1440
cccgaaggac cgcgcacctg gtgcatgacc cgcaagcccg gtgcctga 1488

<210> 4
<211> 1362
<212> DNA
<213> Artificial Sequence

<220>

<223> Inducing factor

<400> 4

atggagagct ctggcaagat ggagagtgga gccggccagc agccgcagcc cccgcagccc 60
ttcctgcctc ccgcagcctg cttctttgcg accgcggcgg cggcggcagc ggcggcggcc 120
gcggcagctc agagcgcgca gcagcaacag ccgcaggcgc cgccgcagca ggcgccgcag 180
ctgagcccgg tggccgacag ccagccctca gggggcggtc acaagtcagc ggccaagcag 240
gtcaagcgcc agcgctcgtc ctctccggaa ctgatgcgct gcaaacgccg gctcaacttc 300
agcggcttcg gctacagcct gccacagcag cagccggccg ccgtggcgcg ccgcaacgag 360
cgcgagcgca accgggtcaa gttggtcaac ctgggttttg ccaccctccg ggagcatgtc 420
cccaacggcg cggccaacaa gaagatgagc aaggtggaga cgctgcgctc ggcggtcgag 480
tacatccgcg cgctgcagca gctgctggac gagcacgacg cggtgagcgc tgcctttcag 540
gcgggcgtcc tgtcgcccac catctccccc aactactcca acgacttgaa ctctatggcg 600
ggttctccgg tctcgtccta ctcctccgac gagggatcct acgaccctct tagcccagag 660
gaacaagagc tgctggactt taccaactgg ttcggctccg gcgagggcag gggaagtctt 720
ctaacatgcg gggacgtgga ggaaaatccc ggcccactcg agatgaccga gtacaagccc 780
acggtgcgcc tcgccacccg cgacgacgtc cccagggccg tacgcaccct cgccgccgcg 840
ttcgccgact accccgccac gcgccacacc gtcgatccgg accgccacat cgagcgggtc 900
accgagctgc aagaactctt cctcacgcgc gtcgggctcg acatcggcaa ggtgtgggtc 960
gcggacgacg gcgccgcggt ggcggtctgg accacgccgg agagcgtcga agcgggggcg 1020
gtgttcgccg agatcggccc gcgcatggcc gagttgagcg gttcccggct ggccgcgcag 1080
caacagatgg aaggcctcct ggcgccgcac cggcccaagg agcccgcgtg gttcctggcc 1140
accgtcggcg tctcgcccga ccaccagggc aagggtctgg gcagcgccgt cgtgctcccc 1200
ggagtggagg cggccgagcg cgccggggtg cccgccttcc tggagacctc cgcgccccgc 1260
aacctcccct tctacgagcg gctcggcttc accgtcaccg ccgacgtcga ggtgcccgaa 1320
ggaccgcgca cctggtgcat gacccgcaag cccggtgcct ga 1362

<210> 5
<211> 1665
<212> DNA
<213> Artificial Sequence

<220>
<223> Inducing factor

<400> 5

atgactggag tctttgacag tctggtggct gatatgcact cgacccagat caccgcctcc 60
agcacgtacc accagcacca gcagcccccg agcggtgcgg gcgccggccc tggcggcaac 120
agcaacagca gcagcagcaa cagcagcctg cacaagcccc aggagtcgcc aaccctcccg 180
gtgtccacgg ctacggacag cagctactac accaaccagc agcacccggc gggcggcggc 240
ggcggggggg cctcgcccta cgcgcacatg ggctcctacc agtaccacgc cagcggcctc 300
aacaatgtct cctactccgc caaaagcagc tacgacctgg gctacaccgc cgcgtacacc 360
tcctacgcgc cctacggcac cagttcgtct ccggtcaaca acgagccgga caaggaagac 420
cttgagcctg aaatccgaat agtgaacggg aagccaaaga aagtccggaa accacgcacc 480
atctactcca gtttccagct ggcggccctt caacgacgct tccagaagac ccagtatctg 540
gccctgccag agcgagccga gctggcggcg tccctgggcc tcacccaaac tcaggtcaaa 600
atctggttcc agaaccgccg atccaagttc aagaagatgt ggaaaagcgg cgagataccc 660
accgagcagc accctggagc cagcgcttct cctccttgtg cctccccgcc ggtctcggcg 720
ccagcatcct gggacttcgg cgcgccgcag cggatggctg gcggcggccc gggcagcgga 780
ggcggcggtg cgggcagctc tggctccagc ccgagcagcg ccgcctcggc ctttctggga 840
aactacccgt ggtaccacca ggcttcgggc tccgcttcac acctgcaggc cacagcgcca 900
cttctgcatc cttcgcagac tccgcaggcg caccatcacc accatcacca ccaccacgca 960
ggcgggggcg ccccggtgag cgcggggacg attttcggct ccggcgaggg caggggaagt 1020
cttctaacat gcggggacgt ggaggaaaat cccggcccac tcgagatgac cgagtacaag 1080
cccacggtgc gcctcgccac ccgcgacgac gtccccaggg ccgtacgcac cctcgccgcc 1140
gcgttcgccg actaccccgc cacgcgccac accgtcgatc cggaccgcca catcgagcgg 1200
gtcaccgagc tgcaagaact cttcctcacg cgcgtcgggc tcgacatcgg caaggtgtgg 1260
gtcgcggacg acggcgccgc ggtggcggtc tggaccacgc cggagagcgt cgaagcgggg 1320
gcggtgttcg ccgagatcgg cccgcgcatg gccgagttga gcggttcccg gctggccgcg 1380
cagcaacaga tggaaggcct cctggcgccg caccggccca aggagcccgc gtggttcctg 1440
gccaccgtcg gcgtctcgcc cgaccaccag ggcaagggtc tgggcagcgc cgtcgtgctc 1500

cccggagtgg aggcggccga gcgcgccggg gtgcccgcct tcctggagac ctccgcgccc 1560
cgcaacctcc ccttctacga gcggctcggc ttcaccgtca ccgccgacgt cgaggtgccc 1620
gaaggaccgc gcacctggtg catgacccgc aagcccggtg cctga 1665

<210> 6
<211> 2085
<212> DNA
<213> Artificial Sequence

<220>
<223> Inducing factor

<400> 6

atgactggag tctttgacag tctggtggct gatatgcact cgacccagat caccgcctcc 60
agcacgtacc accagcacca gcagcccccg agcggtgcgg gcgccggccc tggcggcaac 120
agcaacagca gcagcagcaa cagcagcctg cacaagcccc aggagtcgcc aaccctcccg 180
gtgtccacgg ctacggacag cagctactac accaaccagc agcacccggc gggcggcggc 240
ggcggggggg cctcgcccta cgcgcacatg ggctcctacc agtaccacgc cagcggcctc 300
aacaatgtct cctactccgc caaaagcagc tacgacctgg gctacaccgc cgcgtacacc 360
tcctacgcgc cctacggcac cagttcgtct ccggtcaaca acgagccgga caaggaagac 420
cttgagcctg aaatccgaat agtgaacggg aagccaaaga aagtccggaa accacgcacc 480
atctactcca gtttccagct ggcggccctt caacgacgct tccagaagac ccagtatctg 540
gccctgccag agcgagccga gctggcggcg tccctgggcc tcacccaaac tcaggtcaaa 600
atctggttcc agaaccgccg atccaagttc aagaagatgt ggaaaagcgg cgagataccc 660
accgagcagc accctggagc cagcgcttct cctccttgtg cctccccgcc ggtctcggcg 720
ccagcatcct gggacttcgg cgcgccgcag cggatggctg gcggcggccc gggcagcgga 780
ggcggcggtg cgggcagctc tggctccagc ccgagcagcg ccgcctcggc ctttctggga 840
aactacccgt ggtaccacca ggcttcgggc tccgcttcac acctgcaggc cacagcgcca 900
cttctgcatc cttcgcagac tccgcaggcg caccatcacc accatcacca ccaccacgca 960
ggcgggggcg ccccggtgag cgcggggacg attttcggct ccggcgaggg caggggaagt 1020
cttctaacat gcggggacgt ggaggaaaat cccggcccaa tgaaaaagcc tgaactcacc 1080
gcgacgtctg tcgagaagtt tctgatcgaa aagttcgaca gcgtctccga cctgatgcag 1140
ctctcggagg gcgaagaatc tcgtgctttc agcttcgatg taggagggcg tggatatgtc 1200
ctgcgggtaa atagctgcgc cgatggtttc tacaaagatc gttatgttta tcggcacttt 1260
gcatcggccg cgctcccgat tccggaagtg cttgacattg gggaatttag cgagagcctg 1320
acctattgca tctcccgccg tgcacagggt gtcacgttgc aagacctgcc tgaaaccgaa 1380
ctgcccgctg ttctgcagcc ggtcgcggag gccatggatg cgatcgctgc ggccgatctt 1440
agccagacga gcgggttcgg cccattcgga ccgcaaggaa tcggtcaata cactacatgg 1500
cgtgatttca tatgcgcgat tgctgatccc catgtgtatc actggcaaac tgtgatggac 1560
gacaccgtca gtgcgtccgt cgcgcaggct ctcgatgagc tgatgctttg ggccgaggac 1620
tgccccgaag tccggcacct cgtgcacgcg gatttcggct ccaacaatgt cctgacggac 1680
aatggccgca taacagcggt cattgactgg agcgaggcga tgttcgggga ttcccaatac 1740
gaggtcgcca acatcttctt ctggaggccg tggttggctt gtatggagca gcagacgcgc 1800
tacttcgagc ggaggcatcc ggagcttgca ggatcgccgc ggctccgggc gtatatgctc 1860
cgcattggtc ttgaccaact ctatcagagc ttggttgacg gcaatttcga tgatgcagct 1920
tgggcgcagg gtcgatgcga cgcaatcgtc cgatccggag ccgggactgt cgggcgtaca 1980
caaatcgccc gcagaagcgc ggccgtctgg accgatggct gtgtagaagt actcgccgat 2040
agtggaaacc gacgccccag cactcgtccg agggcaaagg aatag 2085

<210> 7
<211> 1464
<212> DNA
<213> Artificial Sequence

<220>
<223> Inducing factor

<400> 7

atgactggag tctttgacag tctggtggct gatatgcact cgacccagat caccgcctcc 60
agcacgtacc accagcacca gcagcccccg agcggtgcgg gcgccggccc tggcggcaac 120
agcaacagca gcagcagcaa cagcagcctg cacaagcccc aggagtcgcc aaccctcccg 180

gtgtccacgg ctacggacag cagctactac accaaccagc agcacccggc gggcggcggc 240
ggcggggggg cctcgcccta cgcgcacatg ggctcctacc agtaccacgc cagcggcctc 300
aacaatgtct cctactccgc caaaagcagc tacgacctgg gctacaccgc cgcgtacacc 360
tcctacgcgc cctacggcac cagttcgtct ccggtcaaca acgagccgga caaggaagac 420
cttgagcctg aaatccgaat agtgaacggg aagccaaaga aagtccggaa accacgcacc 480
atctactcca gtttccagct ggcggccctt caacgacgct tccagaagac ccagtatctg 540
gccctgccag agcgagccga gctggcggcg tccctgggcc tcacccaaac tcaggtcaaa 600
atctggttcc agaaccgccg atccaagttc aagaagatgt ggaaaagcgg cgagataccc 660
accgagcagc accctggagc cagcgcttct cctccttgtg cctccccgcc ggtctcggcg 720
ccagcatcct gggacttcgg cgcgccgcag cggatggctg gcggcggccc gggcagcgga 780
ggcggcggtg cgggcagctc tggctccagc ccgagcagcg ccgcctcggc ctttctggga 840
aactacccgt ggtaccacca ggcttcgggc tccgcttcac acctgcaggc cacagcgcca 900
cttctgcatc cttcgcagac tccgcaggcg caccatcacc accatcacca ccaccacgca 960
ggcgggggcg ccccggtgag cgcggggacg attttcggct ccggcgaggg caggggaagt 1020
cttctaacat gcggggacgt ggaggaaaat cccggcccat ctagaatggc caagcctttg 1080
tctcaagaag aatccaccct cattgaaaga gcaacggcta caatcaacag catccccatc 1140
tctgaagact acagcgtcgc cagcgcagct ctctctagcg acggccgcat cttcactggt 1200
gtcaatgtat atcattttac tgggggacct tgtgcagaac tcgtggtgct gggcactgct 1260
gctgctgcgg cagctggcaa cctgacttgt atcgtcgcga tcggaaatga gaacaggggc 1320
atcttgagcc cctgcggacg gtgccgacag gtgcttctcg atctgcatcc tgggatcaaa 1380
gccatagtga aggacagtga tggacagccg acggcagttg ggattcgtga attgctgccc 1440
tctggttatg tgtgggaggg ctaa 1464

<210> 8
<211> 2606
<212> DNA
<213> Artificial Sequence

<220>
<223> Inducing factor

<400> 8

atgactggag tctttgacag tctggtggct gatatgcact cgacccagat caccgcctcc 60
agcacgtacc accagcacca gcagcccccg agcggtgcgg gcgccggccc tggcggcaac 120
agcaacagca gcagcagcaa cagcagcctg cacaagcccc aggagtcgcc aaccctcccg 180
gtgtccacgg ctacggacag cagctactac accaaccagc agcacccggc gggcggcggc 240
ggcggggggg cctcgcccta cgcgcacatg ggctcctacc agtaccacgc cagcggcctc 300
aacaatgtct cctactccgc caaaagcagc tacgacctgg gctacaccgc cgcgtacacc 360
tcctacgcgc cctacggcac cagttcgtct ccggtcaaca acgagccgga caaggaagac 420
cttgagcctg aaatccgaat agtgaacggg aagccaaaga aagtccggaa accacgcacc 480
atctactcca gtttccagct ggcggccctt caacgacgct tccagaagac ccagtatctg 540
gccctgccag agcgagccga gctggcggcg tccctgggcc tcacccaaac tcaggtcaaa 600
atctggttcc agaaccgccg atccaagttc aagaagatgt ggaaaagcgg cgagataccc 660
accgagcagc accctggagc cagcgcttct cctccttgtg cctccccgcc ggtctcggcg 720
ccagcatcct gggacttcgg cgcgccgcag cggatggctg gcggcggccc gggcagcgga 780
ggcggcggtg cgggcagctc tggctccagc ccgagcagcg ccgcctcggc ctttctggga 840
aactacccgt ggtaccacca ggcttcgggc tccgcttcac acctgcaggc cacagcgcca 900
cttctgcatc cttcgcagac tccgcaggcg caccatcacc accatcacca ccaccacgca 960
ggcgggggcg ccccggtgag cgcggggacg attttctaac tccccccccc ctaacgttac 1020
tggccgaagc cgcttggaat aaggccggtg tgcgtttgtc tatatgttat tttccaccat 1080
attgccgtct tttggcaatg tgagggcccg gaaacctggc cctgtcttct tgacgagcat 1140
tcctaggggt ctttcccctc tcgccaaagg aatgcaaggt ctgttgaatg tcgtgaagga 1200
agcagttcct ctggaagctt cttgaagaca aacaacgtct gtagcgaccc tttgcaggca 1260
gcggaacccc ccacctggcg acaggtgcct ctgcggccaa aagccacgtg tataagatac 1320
acctgcaaag gcggcacaac cccagtgcca cgttgtgagt tggatagttg tggaaagagt 1380
caaatggctc tcctcaagcg tattcaacaa ggggctgaag gatgcccaga aggtacccca 1440
ttgtatggga tctgatctgg ggcctcggtg cacatgcttt acatgtgttt agtcgaggtt 1500
aaaaaaacgt ctaggccccc cgaaccacgg ggacgtggtt ttcctttgaa aaacacgatg 1560
ataatatggc cacagctaga atgaaaaagc ctgaactcac cgcgacgtct gtcgagaagt 1620
ttctgatcga aaagttcgac agcgtctccg acctgatgca gctctcggag ggcgaagaat 1680
ctcgtgcttt cagcttcgat gtaggagggc gtggatatgt cctgcgggta aatagctgcg 1740
ccgatggttt ctacaaagat cgttatgttt atcggcactt tgcatcggcc gcgctcccga 1800

ttccggaagt gcttgacatt ggggaattta gcgagagcct gacctattgc atctcccgcc 1860
gtgcacaggg tgtcacgttg caagacctgc ctgaaaccga actgcccgct gttctgcagc 1920
cggtcgcgga ggccatggat gcgatcgctg cggccgatct tagccagacg agcgggttcg 1980
gcccattcgg accgcaagga atcggtcaat acactacatg gcgtgatttc atatgcgcga 2040
ttgctgatcc ccatgtgtat cactggcaaa ctgtgatgga cgacaccgtc agtgcgtccg 2100
tcgcgcaggc tctcgatgag ctgatgcttt gggccgagga ctgccccgaa gtccggcacc 2160
tcgtgcacgc ggatttcggc tccaacaatg tcctgacgga caatggccgc ataacagcgg 2220
tcattgactg gagcgaggcg atgttcgggg attcccaata cgaggtcgcc aacatcttct 2280
tctggaggcc gtggttggct tgtatggagc agcagacgcg ctacttcgag cggaggcatc 2340
cggagcttgc aggatcgccg cggctccggg cgtatatgct ccgcattggt cttgaccaac 2400
tctatcagag cttggttgac ggcaatttcg atgatgcagc ttgggcgcag ggtcgatgcg 2460
acgcaatcgt ccgatccgga gccgggactg tcgggcgtac acaaatcgcc cgcagaagcg 2520
cggccgtctg gaccgatggc tgtgtagaag tactcgccga tagtggaaac cgacgcccca 2580
gcactcgtcc gagggcaaag gaatag 2606

<210> 9
<211> 1452
<212> DNA
<213> Artificial Sequence

<220>
<223> Inducing factor

<400> 9

atgactggag tctttgacag tctagtggct gatatgcact cgacccagat cgccgcctcc 60
agcacgtacc accagcacca gcagcccccg agcggcggcg gcgccggccc gggtggcaac 120
agcagcagca gcagcagcct ccacaagccc caggagtcgc ccacccttcc ggtgtccacc 180
gccaccgaca gcagctacta caccaaccag cagcacccgg cgggcggcgg cggcggcggg 240
ggctcgccct acgcgcacat gggttcctac cagtaccaag ccagcggcct caacaacgtc 300
ccttactccg ccaagagcag ctatgacctg ggctacaccg ccgcctacac ctcctacgct 360
ccctatggaa ccagttcgtc cccagccaac aacgagcctg agaaggagga ccttgagcct 420
gaaattcgga tagtgaacgg gaagccaaag aaagtccgga aaccccgcac catctactcc 480
agtttccagc tggcggctct tcagcggcgt ttccaaaaga ctcaatactt ggccttgccg 540
gagcgagccg agctggcggc ctctctgggc ctcacccaga ctcaggtcaa aatctggttc 600
cagaaccgcc ggtccaagtt caagaagatg tggaaaagtg gtgagatccc ctcggagcag 660
caccctgggg ccagcgcttc tccaccttgt gcttcgccgc cagtctcagc gccggcctcc 720
tgggactttg gtgtgccgca gcggatggcg ggcggcggtg gtccgggcag tggcggcagc 780
ggcgccggca gctcgggctc cagcccgagc agcgcggcct cggcttttct gggcaactac 840
ccctggtacc accagacctc gggatccgcc tcacacctgc aggccacggc gccgctgctg 900
caccccactc agaccccgca gccgcatcac caccaccacc atcacggcgg cgggggcgcc 960
ccggtgagcg cggggacgat tttcggctcc ggcgagggca ggggaagtct tctaacatgc 1020
ggggacgtgg aggaaaatcc cggcccatct agaatggcca agcctttgtc tcaagaagaa 1080
tccaccctca ttgaaagagc aacggctaca atcaacagca tccccatctc tgaagactac 1140
agcgtcgcca gcgcagctct ctctagcgac ggccgcatct tcactggtgt caatgtatat 1200
cattttactg ggggaccttg tgcagaactc gtggtgctgg gcactgctgc tgctgcggca 1260
gctggcaacc tgacttgtat cgtcgcgatc ggaaatgaga acaggggcat cttgagcccc 1320
tgcggacggt gccgacaggt gcttctcgat ctgcatcctg ggatcaaagc catagtgaag 1380
gacagtgatg gacagccgac ggcagttggg attcgtgaat tgctgccctc tggttatgtg 1440
tgggagggct aa 1452

<210> 10
<211> 1377
<212> DNA
<213> Artificial Sequence

<220>
<223> Inducing factor

<400> 10

atggaaagct ctgccaagat ggagagcggc ggcgccggcc agcagcccca gccgcagccc 60
cagcagccct tcctgccgcc cgcagcctgt ttctttgcca cggccgcagc cgcggcggcc 120

gcagccgccg cagcggcagc gcagagcgcg cagcagcagc agcagcagca gcagcagcag 180
cagcaggcgc cgcagctgag accggcggcc gacggccagc cctcaggggg cggtcacaag 240
tcagcgccca agcaagtcaa gcgacagcgc tcgtcttcgc ccgaactgat gcgctgcaaa 300
cgccggctca acttcagcgg ctttggctac agcctgccgc agcagcagcc ggccgccgtg 360
gcgcgccgca acgagcgcga gcgcaaccgc gtcaagttgg tcaacctggg ctttgccacc 420
cttcgggagc acgtccccaa cggcgcggcc aacaagaaga tgagtaaggt ggagacactg 480
cgctcggcgg tcgagtacat ccgcgcgctg cagcagctgc tggacgagca tgacgcggtg 540
agcgccgcct tccaggcagg cgtcctgtcg cccaccatct cccccaacta ctccaacgac 600
ttgaactcca tggccggctc gccggtctca tcctactcgt cggacgaggg ctcttacgac 660
ccgctcagcc ccgaggagca ggagcttctc gacttcacca actggttcgg ctccggcgag 720
ggcaggggaa gtcttctaac atgcggggac gtggaggaaa atcccggccc actcgagatg 780
accgagtaca agcccacggt gcgcctcgcc acccgcgacg acgtccccag ggccgtacgc 840
accctcgccg ccgcgttcgc cgactacccc gccacgcgcc acaccgtcga tccggaccgc 900
cacatcgagc gggtcaccga gctgcaagaa ctcttcctca cgcgcgtcgg gctcgacatc 960
ggcaaggtgt gggtcgcgga cgacggcgcc gcggtggcgg tctggaccac gccggagagc 1020
gtcgaagcgg gggcggtgtt cgccgagatc ggcccgcgca tggccgagtt gagcggttcc 1080
cggctggccg cgcagcaaca gatggaaggc ctcctggcgc cgcaccggcc caaggagccc 1140
gcgtggttcc tggccaccgt cggcgtctcg cccgaccacc agggcaaggg tctgggcagc 1200
gccgtcgtgc tccccggagt ggaggcggcc gagcgcgccg gggtgcccgc cttcctggag 1260
acctccgcgc cccgcaacct ccccttctac gagcggctcg gcttcaccgt caccgccgac 1320
gtcgaggtgc ccgaaggacc gcgcacctgg tgcatgaccc gcaagcccgg tgcctga 1377

## Description

### FIELD

The present invention relates to cell technology, and to a nerve cell production method.

### BACKGROUND

Induced pluripotent stem cells (iPS cells) are capable of transforming into numerous types of cells composing the body. Therefore, iPS cells capable of transforming into various types of somatic cells or tissues are considered promising for use in cell graft therapy and innovative drug development and research. In 2014, for example, retina cells produced from iPS cells were successfully applied in transplantation therapy. Projects are being pursued not only in Japan but throughout the world, for creating brain cells and different organ cells from iPS cells for use in transplantation therapy.

Numerous methods for altering iPS cells to differentiated cells exist in the prior art. For utilization of iPS cells in transplantation therapy, however, it is important to establish highly efficient differentiation-inducing methods for iPS cells. Specifically, it is necessary to establish techniques to be used for inducing differentiation of iPS cells to differentiated cells, improving the differentiation-inducing efficiency and precision and ensuring that the functionality of the created differentiated cells is able to withstand transplantation therapy.

Methods for inducing differentiated cells from iPS cells or embryonic stem cells (ES cells) to somatic cells have conventionally included methods that imitate the process of development, by combining hormones or growth factors that are the determinants of the properties of the cells, as well as low molecular compounds, and varying their quantity ratios or concentrations with time. However, it is difficult to completely emulate the development process *in vitro,* and efficiency is also poor. Moreover, inducing differentiation of human somatic cells requires a much longer differentiation-inducing period than for mice, with 3 months or longer, for example, being necessary to prepare mature nerves.

Another problem is that differentiation-inducing efficiency differs widely depending on the type of ES/iPS cells, while the properties of induced somatic cells are non-homogeneous. When chemical substances have actually been added to different types of ES cell clones to create various types of cells, it has been demonstrated that certain clones exist that readily differentiate to pancreas cells or that readily differentiate to heart cells, and therefore that different clones have varying differentiation potencies (see NPL 1, for example). In addition, it has been demonstrated that when the method known as a serum-free suspension culture method (SFEBq method) is used, in which iPS cells are cultured in medium free of serum or of chemical substances that inhibit neuron differentiation, to produce neurons from iPS cells/ES cells, thereby producing neurons from dozens of types of iPS cells, some of the iPS/ES cell clones present are difficult to transform into neurons (see NPL 2, for example).

Specifically, cells whose differentiation has been induced from human ES/iPS cells by methods utilizing hormones or chemical substances have been confirmed to be fetal-stage somatic cells in the initial stages. It is extremely difficult to induce differentiation of mature human somatic cells, and their culturing requires long periods of several months. However, for innovative drug development and transplant medicine for fully developed individuals, it is important to prepare somatic cells that match the maturation level of the individual.

For neurons, which include cells of a variety of different subtypes, it is not possible to induce differentiation of neuronal subtypes in a uniform manner from ES/iPS cells by methods utilizing hormones or chemical substances. Therefore, innovative drug screening specific for designated neuronal subtypes is not possible. This lowers the efficiency for innovative drug screening. For transplant medicine as well, it is not possible to concentrate and transplant only specific diseased cells.

For this reason, methods have been proposed wherein genes for the properties of specific somatic cells are directly transferred into ES/iPS cells using viruses, to create the desired somatic cells. Methods using viruses allow specific creation of mature neurons in very short time periods compared to methods using hormones or chemical substances, such as 2 weeks, for example. Moreover, creating neurons by specific gene transfer allows excitatory nerves alone, for example, to be obtained in a homogeneous manner. Therefore, specific innovative drug screening for specific neuronal subtypes becomes possible, potentially making it possible to concentrate and transplant only cells specific to a disease, for transplant medicine.

However, in methods of inducing differentiation of stem cells to somatic cells using viruses to cause expression of specific genes, the genes are inserted in the genome of the ES/iPS cells, causing damage to the endogenous genes. This has resulted in problems such as failure to properly accomplish innovative drug screening, and the risk of canceration of grafts (see NPLs 3 and 4, for example).

### [Citation List]

### [Non-patent literature]

NPL 1: Nature Biotechnol 26(3): 313-315, 2008.
NPL 2: PNAS, 111:12426-12431, 2014
NPL 3: N Eng J Med, 346:1185-1193, 2002
NPL 4: Science 302: 415-419, 2003

### SUMMARY

### [TECHNICAL PROBLEM]

It is an object of the present invention to provide a nerve cell production method that allows nerve cells to be produced efficiently in a short period of time, without damaging cellular genes.

### [SOLUTION TO PROBLEM]

According to one aspect of the invention there is provided a nerve cell production method that includes preparing stem cells, introducing inducing factor RNA into the stem cells and causing their differentiation into nerve cells.

In this nerve cell production method, the stem cells may be induced pluripotent stem cells. Nerve cells may be neurons, neural stem cells or neural precursor cells. Neurons may be inhibitory neurons, excitatory neurons or dopamine-producing neurons. Alternatively, nerve cells may be motor nerve cells, oligodendrocyte progenitor cells or oligodendrocytes.

In the nerve cell production method, the inducing factor RNA may be introduced into the stem cells by a lipofection method.

In the nerve cell production method, the inducing factor RNA may include mRNA corresponding to a drug resistance gene.

The nerve cell production method may also include, after introducing the inducing factor RNA into the stem cells, selecting cells that exhibit drug resistance.

According to another aspect of the invention there is provided a nerve cell production method that includes preparing cells, introducing reprogramming factor RNA into the cells, and introducing inducing factor RNA into the cells in which the reprogramming factor RNA has been introduced, to cause their differentiation into nerve cells. Conventionally, it has taken 2 months to establish stem cells and 3 months to induce nerve cells. With the method for producing nerve cells according to this aspect of the invention, however it is possible to induce nerve cells from cells in a shorter period of time.

In this nerve cell production method, reprogramming factor RNA may be introduced into cells and inducing factor RNA may be introduced into the reprogramming factor RNA-introduced cells, all in the same culturing vessel.

Furthermore, in this nerve cell production method, after the reprogramming factor RNA has been introduced into the cells, the inducing factor RNA may be introduced into the reprogramming factor RNA-introduced cells without detaching the reprogramming factor RNA-introduced cells from the culturing vessel.

Alternatively, in this nerve cell production method, after the reprogramming factor RNA has been introduced into the cells, the inducing factor RNA may be introduced into the reprogramming factor RNA-introduced cells after detaching the reprogramming factor RNA-introduced cells from the culturing vessel and seeding the reprogramming factor RNA-introduced cells into a different culturing vessel.

In this nerve cell production method, the cells into which the reprogramming factor RNA is to be introduced may be somatic cells such as human fibroblasts or blood cells.

Nerve cells for this nerve cell production method may be neurons, neural stem cells or neural precursor cells. Neurons may be inhibitory neurons, excitatory neurons or dopamine-producing neurons.

In the nerve cell production method, the inducing factor RNA may be introduced into the stem cells by a lipofection method.

In the nerve cell production method, the inducing factor RNA may include mRNA corresponding to the drug resistance gene.

The nerve cell production method may also include, after introducing the inducing factor RNA into the stem cells, selecting cells that exhibit drug resistance.

According to another aspect of the invention, there is provided RNA corresponding to DNA of any one of SEQ ID NO: 1 to 10.

According to another aspect of the invention, there is provided an inducing factor comprising RNA corresponding to DNA of any one of SEQ ID NO: 1 to 10.

### [ADVANTAGEOUS EFFECTS OF INVENTION]

According to the invention it is possible to provide a nerve cell production method that allows nerve cells to be produced efficiently in a short period of time, without damaging cellular genes.

### BRIEF DESCRIPTION OF DRAWINGS

Fig. 1 is a photograph of neurons in Example 1 of the first embodiment.
Fig. 2 is a photograph of neurons in Example 1 of the first embodiment.
Fig. 3 is a photograph of neurons in Example 1 of the first embodiment.
Fig. 4 is a photograph of neurons in Examples 2 to 4 of the first embodiment.
Fig. 5 is a photograph of neurons in Example 3 of the first embodiment.
Fig. 6 is a photograph of neurons in Example 3 of the first embodiment.
Fig. 7 is a photograph of neurons in Example 5 of the first embodiment.
Fig. 8 is a photograph of neurons in Example 6 of the first embodiment.
Fig. 9 is a photograph of neurons in Example 7 of the first embodiment.
Fig. 10 is a photograph of neurons in Example 8 of the first embodiment.
Fig. 11 is a photograph of neurons in Comparative Example 1 of the first embodiment.
Fig. 12 is a table showing the reprogramming factor mRNA master mix components used in Example 1 of a second embodiment.
Fig. 13 is a table showing the contents of the kit used in Example 1 of the second embodiment.
Fig. 14 is a photograph of neurons in Example 1 of the second embodiment.
Fig. 15 is a photograph of neurons in Example 2 of the second embodiment.
Fig. 16 is a photograph of neurons in Example 2 of the second embodiment.

### DESCRIPTION OF EMBODIMENTS

Embodiments of the invention will now be explained in detail. The embodiments described below are merely examples of devices and methods for implementing the technical concept of the invention, and the technical concept of the invention is not limited to the described combinations of structural members. The technical concept of the invention may incorporate various modifications such as are within the scope of the Claims.

### (First embodiment)

The nerve cell production method according to the first embodiment includes preparing stem cells, introducing inducing factor RNA into the stem cells and causing their differentiation into nerve cells.

Both induced pluripotent stem cells (iPS cells) and embryonic stem cells (ES cells) may be used as stem cells.

Examples of nerve cells to be induced include neurons, neural stem cells and neural precursor cells. Examples of neurons include inhibitory neurons, excitatory neurons and dopamine-producing neurons. Alternatively, nerve cells may be motor nerve cells, oligodendrocyte progenitor cells or oligodendrocytes.

The culture solution used for culturing of the stem cells may be Primate ES Cell Medium, mTeSR1, TeSR2, TeSRE8 (Stemcell Technologies), or the like.

The medium for culturing the stem cells may also include a gel. The gel may include one or more high molecular compounds selected from the group consisting of deacylated gellan gum, gellan gum, hyaluronic acid, rhamsan gum, diutan gum, xanthan gum, carrageenan, fucoidan, pectin, pectic acid, pectinic acid, heparan sulfate, heparin, heparitin sulfate, keratosulfate, chondroitin sulfate, dermatan sulfate, rhamnan sulfate, and salts of the foregoing. The gel medium may also include methyl cellulose. Including methyl cellulose allows greater control of aggregation between the cells.

The gel may also be a temperature-sensitive gel. The temperature-sensitive gel may include at least one type selected from among poly(glycerol monomethacrylate) (PGMA), poly(2-hydroxypropyl methacrylate) (PHPMA), poly (N-isopropylacrylamide) (PNIPAM), amine terminated, carboxylic acid terminated, maleimide terminated, N-hydroxysuccinimide (NHS) ester terminated, triethoxysilane terminated, poly (N-isopropylacrylamide-co-acrylamide), poly (N-isopropylacrylamide-co-acrylic acid), poly (N-isopropylacrylamide-co-butylacrylate), poly (N-isopropylacrylamide-co-methacrylic acid), poly (N-isopropylacrylamide-co-methacrylic acid-co-octadecyl acrylate) and N-isopropylacrylamide.

The medium for culturing of the stem cells may also include one or more substances selected from the group consisting of cadherin, laminin, fibronectin and vitronectin.

The inducing factor RNA to be introduced into the stem cells may include any one or more from among ASCL1 (Achaete-Scute Homolog 1) mRNA, DLX2 (Distal-Less Homeobox 2) mRNA, MYT1L (Myelin Transcription Factor 1-Like) mRNA and NGN2 (neurogenin 2) mRNA. The gene symbols used here refer to human genes, but there is no intention to restrict the species by the use of uppercase or lowercase symbols. For example, even if all of the symbols are uppercase, this is not intended to exclude genes of mice or rats. In the Examples, however, the gene symbols given are according to the actual biological species used.

The inducing factor RNA may include mRNA corresponding to a drug resistance gene. A "drug" is, for example, an antibiotic such as puromycin, neomycin, blasticidin, G418, hygromycin or zeocin. The cells into which the inducing factor RNA has been introduced exhibit drug resistance.

The mRNA in the inducing factor RNA may be modified with one or more selected from the group consisting of pseudouridine (Ψ), 5-methyluridine (5meU), N1-methylpseudouridine (me1Ψ), 5-methoxyuridine (5moU), 5-hydroxymethyluridine (5hmU), 5-formyluridine (5fU), 5-carboxymethyl esteruridine (5camU), thienoguanosine (thG), N4-methylcytidine (me4C), 5-methylcytidine (m5C), 5-methoxycytidine (5 moC), 5-hydroxymethylcytidine (5hmC), 5-hydroxycytidine (5hoC), 5-formylcytidine (5fC), 5-carboxycytidine (5caC), N6-methyl-2-aminoadenosine m6DAP), diaminopurine (DAP), 5-methyluridine (m5U), 2'-O-methyluridine (Um or m2'-OU), 2-thiouridine (s2U) and N6-methyladenosine (m6A).

The mRNA may also be polyadenylated. The mRNA may be prepared by polyadenylation of (IVT)RNA that is transcribed *in vitro.* The mRNA may also be polyadenylated during IVT, using a DNA template coding for poly(A) ends. The mRNA may also be capped. Most of the mRNA molecules may be given caps to maximize expression efficiency in the cells.

The mRNA may also have a 5'cap[m7G(5')ppp(5')G] structure. This sequence stabilizes mRNA and promotes transcription. In the case of mRNA containing 5'-triphosphate, the 5'-triphosphate may be removed by dephosphorylation treatment. The mRNA may also have [3'O-Me-m7G(5')ppp(5')G] as an Anti-Reverse Cap Analog (ARCA). ARCA is a sequence inserted before the transcription initiation site, and it doubles the efficiency of the mRNA to be transcribed. The mRNA may also have a PolyA tail.

The inducing factor RNA includes, for example, NGN2-T2A-PURO mRNA (TriLink, RNA corresponding to DNA listed as SEQ ID NO: 1). Cells transfected with NGN2-T2A-PURO mRNA (Trilink) produce neurogenin 2 (NGN2) and exhibit puromycin resistance. The mRNA may be capped with Anti-Reverse Cap Analog (ARCA) and polyadenylated, and optionally substituted with 5-methylcytidine and pseudouridine. The ability of antibody to recognize mRNA is reduced by 5-methylcytidine and pseudouridine. RNA corresponding to the DNA listed as SEQ ID NO: 2 may also be used. The DNA listed as SEQ ID NO: 2 is DNA having the xbal restriction site removed from the DNA of SEQ ID NO: 1.

Alternatively, the inducing factor includes NGN2-T2A-PURO mRNA (RNA corresponding to the DNA listed as SEQ ID NO: 3). Cells transfected with RNA corresponding to the DNA listed as SEQ ID NO: 3 produce NGN2 and exhibit puromycin resistance.

Alternatively, the inducing factor includes ASCL1-T2A-PURO mRNA (RNA corresponding to the DNA listed as SEQ ID NO: 4). Cells transfected with RNA corresponding to the DNA listed as SEQ ID NO: 4 produce ASCL1 and exhibit puromycin resistance.

Alternatively, the inducing factor includes DLX2-T2A-PURO mRNA (RNA corresponding to the DNA listed as SEQ ID NO: 5). Cells transfected with RNA corresponding to the DNA listed as SEQ ID NO: 5 produce DLX2 and exhibit puromycin resistance.

Alternatively, the inducing factor includes DLX2-T2A-HYGRO mRNA (RNA corresponding to the DNA listed as SEQ ID NO: 6). Cells transfected with RNA corresponding to the DNA listed as SEQ ID NO: 6 produce DLX2 and exhibit hygromycin resistance.

Alternatively, the inducing factor includes DLX2-T2A-BLAST mRNA (RNA corresponding to the DNA listed as SEQ ID NO: 7). Cells transfected with RNA corresponding to the DNA listed as SEQ ID NO: 7 produce DLX2 and exhibit blasticidin resistance.

Alternatively, the inducing factor includes DLX2-IRES-HYGRO mRNA (RNA corresponding to the DNA listed as SEQ ID NO: 8). Cells transfected with RNA corresponding to the DNA listed as SEQ ID NO: 8 produce DLX2 and exhibit hygromycin resistance.

Alternatively, the inducing factor includes DLX2-IRES-BLAST mRNA (RNA corresponding to the DNA listed as SEQ ID NO: 9). Cells transfected with RNA corresponding to the DNA listed as SEQ ID NO: 9 produce DLX2 and exhibit blasticidin resistance.

Alternatively, the inducing factor includes ASCL1-T2A-PURO mRNA (RNA corresponding to the DNA listed as SEQ ID NO: 10). Cells transfected with RNA corresponding to the DNA listed as SEQ ID NO: 10 produce ASCL1 and exhibit puromycin resistance.

The inducing factor RNA is introduced into stem cells by a transfection method, such as lipofection, for example. Lipofection is a method in which a complex of nucleic acid as a negatively charged substance with positively charged lipids, is formed by electrical interaction, and the complex is incorporated into cells by endocytosis or membrane fusion. Lipofection is advantageous as it creates little damage to cells and has excellent introduction efficiency, while operation is convenient and less time is required.

Transfection of the inducing factor RNA may be carried out using Lipofectamine MessengerMAX^{R} as the transfection reagent. In addition, the RNA lipofection reagent used may be Lipofectamin^{R} RNAiMAX (Thermo Fisher Scientific), Lipofectamin^{R} 2000, Lipofectamin^{R} 3000, NeonTransfection System (Thermo Fisher Scientific), Stemfect RNA transfection reagent (Stemfect), mRNA-In^{R} (Molecular Transfer, Inc.), NextFect^{R} RNA Transfection Reagent (BioScientific), Amaxa^{R} Human T cell Nucleofector^{R} kit (Lonza, VAPA-1002), Amaxa^{R} Human CD34 cell Nucleofector^{R} kit (Lonza, VAPA-1003), or ReproRNA^{R} transfection reagent Stemcell Technologies).

Transfection of the inducing factor RNA may also be carried out several times.

The medium used for transfection of the inducing factor RNA is, for example, serum-free or low serum medium such as Plurito Reprogramming Medium (Stemgent) or Opti-MEM^{R} (Gibco). The medium used during, and before and after, transfection of the inducing factor RNA may also include B18R protein. B18R protein reduces congenital antiviral reaction of the cells. B18R protein is sometimes used to inhibit cell death due to immunoreaction during insertion of RNA into cells. However, the medium does not need to include B18R protein, or it may contain B18R protein in a low concentration of 0.01% to 1%.

After transfection of the inducing factor RNA, or after several procedures of transfection of the inducing factor RNA, the medium may be exchanged with medium suited for nerve cells.

If the inducing factor RNA included mRNA corresponding to the drug resistance gene, then cells exhibiting drug resistance can be selected either during or after transfection. For example, when the inducing factor RNA includes mRNA corresponding to a puromycin resistance gene, the transfected cells may be exposed to puromycin to kill the cells other than those in which the inducing factor RNA has been introduced, and select out the cells in which the inducing factor RNA has been introduced. The inducing factor RNA may include, as mRNA corresponding to drug resistance genes, any mRNA selected from among neomycin, blasticidin, G418, hygromycin and zeocin.

Differentiation to nerve cells can be confirmed by whether or not they are positive for NGN2, β-III Tubulin, MAP2, PSA-NCAM, vGLUT, GAD67, TH (Tyrosine Hydroxylase), SOX1, SOX2, CD133, Nestin, HB9, ISL1, O4, PLP1, MOG or MBP. NGN2 is a switch protein necessary for neuron differentiation. B-III Tubulin, MAP2 and PSA-NCAM are neuron markers. vGLUT is a marker for excitatory neurons. GAD67 is a marker for inhibitory neurons. TH is a marker for dopamine-producing neurons. SOX1, SOX2, CD133 and Nestin are markers for neural stem cells. HB9 and ISL1 are markers for motor neurons. O4, PLP1, MOG and MBP are markers for oligodendrocyte precursors.

GFAP and CD44 can be used as astrocyte precursor and astrocyte markers. ChAT can be used as a marker for cholinergic neurons.

In the method of the first embodiment described above, RNA coding for a specific gene is expressed in stem cells, allowing efficient creation of nerve cells without damaging the stem cell genes.

In a method of producing nerve cells from stem cells using only hormones or chemical substances, an extremely long time is necessary until the nerve cells are produced. With the method of the first embodiment, however, it is possible to produce nerve cells in a very short period of time.

In a method of producing nerve cells from stem cells using hormones or chemical substances, only some of the stem cells are transformed into the target nerve cells. With the method of the first embodiment, however, at least 90% of the cells into which the inducing factor RNA is introduced are transformed into the target nerve cells.

Moreover, in methods of producing nerve cells from stem cells using hormones or chemical substances, even following the same protocol results in clones that can be used as the target nerve cells and clones that cannot, and therefore variation exists among the clones. In the method of the first embodiment, however, it is possible to obtain high differentiation-inducing efficiency with multiple clones.

When cytokines from an undifferentiated cell population are used to induce differentiation and produce cells to be used for grafting, undifferentiated cells can potentially remain in the cells to be used for grafting. The residual undifferentiated cells can undergo their own cell division and proliferation at the grafting site, posing the risk of forming teratomas. In contrast, in the method of the first embodiment it is possible to simultaneously express a drug resistance gene as well, thus allowing drug selection of cells into which the inducing factor RNA has been introduced. It is thus possible to avoid the risk of undifferentiated cell contamination or teratoma formation, making it suitable for transplant medicine.

### (Example 1 of the first embodiment)

A plate coated with a solubilized basal membrane preparation (Matrigel, Corning) was prepared. After suspending iPS cells dispersed into 1 × 10⁵, 2 × 10⁵ or 4 × 10⁵ single cells in 1 mL of human ES/iPS cell-supporting medium (mTeSR1, TEMCELL Technologies) containing B18R recombinant protein at a concentration of 200 ng/mL and ROCK inhibitor (Y-27632, Selleck), the suspension was added to the plate, seeding the cells, and the suspension was allowed to stand for one day.

The medium was exchanged with 1 mL of xeno-free medium (Pluriton, Stemgent) containing B18R recombinant protein at a concentration of 200 ng/mL and ROCK inhibitor.

A 1.5 mL micro centrifuge tube A and a 1.5 mL micro centrifuge tube B were also prepared.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture.

In tube B there was placed 62.5 µL of low serum medium (Opti-MEM^{R}, Gibco), and then 750 ng of ASCL1 mRNA, 250 ng of DLX2-T2A-BLAST mRNA and 100 ng of GFP mRNA (Trilink) were added and the mixture was thoroughly agitated to obtain a second reaction mixture. ASCL1 is a protein that regulates differentiation to general neurons. DLX2 is a protein that regulates differentiation to inhibitory neurons.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, and 1 mL of xeno-free medium (Pluriton, Stemgent) containing B18R recombinant protein at a concentration of 200 ng/mL and ROCK inhibitor was placed in the plate and allowed to stand overnight at 37°C.

This resulted in transfection of the mRNA into the cells (Day 1), similar to the previous day. All of the medium was then removed from the plate, and 1 mL of xeno-free medium (Pluriton, Stemgent) containing B18R recombinant protein at a concentration of 200 ng/mL and blasticidin as an antibiotic at a concentration of 20 ng/mL, was placed in the plate and allowed to stand overnight at 37°C.

All of the medium was removed from the plate, and 1 mL of xeno-free medium (Pluriton, Stemgent) containing B18R recombinant protein at a concentration of 200 ng/mL and ROCK inhibitor was placed in the plate. This resulted in transfection of the mRNA into the cells (Day 2), similar to the previous days. All of the medium was then removed from the plate, and 1 mL of neuron medium (DMEM/F12, 25 µg/mL insulin, 50 µg/mL human transferrin, 30 nmol/L sodium selenite, 20 nmol/L progesterone and 100 nmol/L putrescine, a neuron medium that will be referred to as "N3 medium") containing B18R recombinant protein at a concentration of 200 ng/mL and blasticidin at a concentration of 20 ng/mL, was placed in the plate and allowed to stand overnight at 37°C.

All of the medium was removed from the plate, and 1 mL of N3 medium containing B18R recombinant protein at a concentration of 200 ng/mL was placed in the plate. This resulted in transfection of the mRNA into the cells (Day 3), similar to the previous days. All of the medium was then removed from the plate, and 1 mL of N3 medium containing B18R recombinant protein at a concentration of 200 ng/mL and blasticidin at a concentration of 20 µg/mL, was placed in the plate and allowed to stand overnight at 37°C.

All of the medium was removed from the plate, and 1 mL of N3 medium containing B18R recombinant protein at a concentration of 200 ng/mL was placed in the plate and allowed to stand overnight at 37°C. The cells were then cultured for 7 days. Selection with blasticidin was carried out up to Day 6.

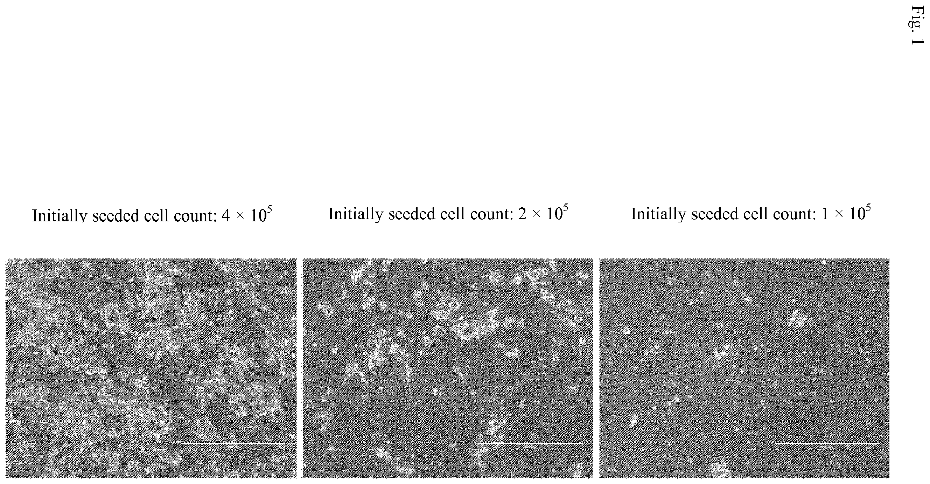

As a result, as shown in Fig. 1, induction of neurons from iPS cells was confirmed based on cell morphology.

The medium was then removed from the plate and the cells were rinsed with PBS. Next, 4% PFA was placed in the plate, and reaction was conducted for 15 minutes at 4°C to fix the cells. The cells were further rinsed twice with PBS, and then the primary antibody was diluted with PBS medium containing 5% CCS and 0.1% Triton and added to the plate. The primary antibody used was GAD67 mouse monoclonal antibody IgG2a (MAB5406, Millipore), as a marker for inhibitory neurons.

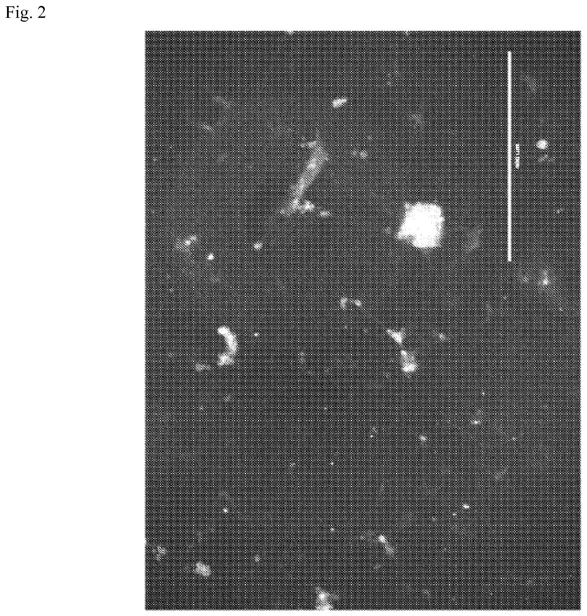

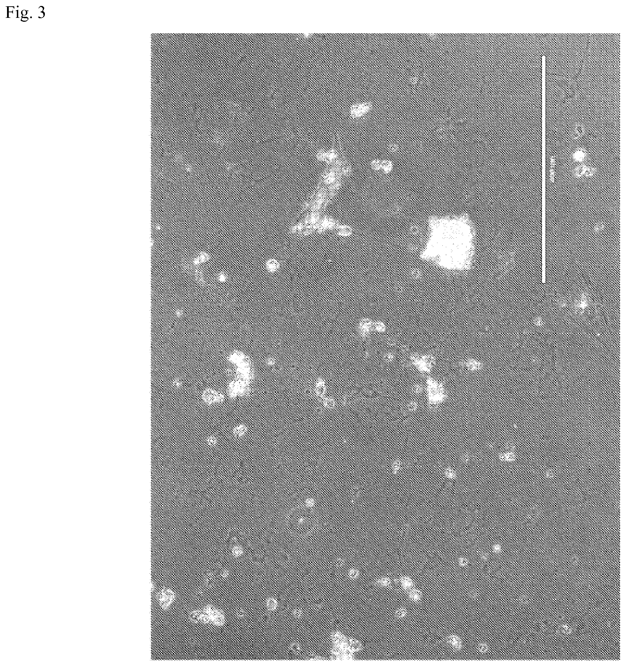

After one hour of reaction at room temperature, PBS was added to the plate and thoroughly mixed with it, and then the PBS was discarded. PBS was again added and discarded, a solution containing fluorescent-labeled donkey anti-mouse IgG (H+L) secondary antibody (Alexa Fluor^{R}, 555, Conjugate, Invitrogen) was added to the plate, and reaction was conducted at room temperature for 30 minutes. The cells were then rinsed twice with PBS and observed under a fluorescent microscope. As a result, as shown in Fig. 2 (fluorescent microscope observation image) and Fig. 3 (phase contrast microscope observation/fluorescent microscope observation merged image), the neurons induced from the iPS cells were confirmed to be expressing GAD67 as a marker of inhibitory neurons.

### (Example 2 of the first embodiment)

A plate coated with a solubilized basal membrane preparation (Matrigel, Corning) was prepared. After suspending single cell-dispersed iPS cells in 1 mL of human ES/iPS cell-supporting medium (mTeSR1, TEMCELL Technologies) containing B18R recombinant protein at a concentration of 200 ng/mL and ROCK inhibitor, the suspension was added to the plate, seeding the cells, and the suspension was allowed to stand for one day.

The medium was exchanged with 1 mL of xeno-free medium (Pluriton, Stemgent) containing B18R recombinant protein at a concentration of 200 ng/mL and ROCK inhibitor.

Also, a 1.5 mL micro centrifuge tube A and a 1.5 mL micro centrifuge tube B were prepared.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture.

In tube B there was placed 62.5 µL of low serum medium (Opti-MEM^{R}, Gibco), and then 500 ng of ASCL1-PURO mRNA, 250 ng of DLX2-T2A-BLAST mRNA and 100 ng of GFP mRNA (Trilink) were added and the mixture was thoroughly agitated to obtain a second reaction mixture.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, and 1 mL of xeno-free medium (Pluriton, Stemgent) containing B18R recombinant protein at a concentration of 200 ng/mL and ROCK inhibitor was placed in the plate and allowed to stand overnight at 37°C.

This resulted in transfection of the mRNA into the cells (Day 1), similar to the previous day. All of the medium was then removed from the plate, and 1 mL of xeno-free medium (Pluriton, Stemgent) containing B18R recombinant protein at a concentration of 200 ng/mL, blasticidin as an antibiotic at a concentration of 20 µg/mL, puromycin at a concentration of 2 µg/mL and ROCK inhibitor, was placed in the plate and allowed to stand overnight at 37°C.

All of the medium was removed from the plate, and 1 mL of xeno-free medium (Pluriton, Stemgent) containing B18R recombinant protein at a concentration of 200 ng/mL and ROCK inhibitor was placed in the plate. This resulted in transfection of the mRNA into the cells (Day 2), similar to the previous days. All of the medium was then removed from the plate, and 1 mL of N3 medium containing B18R recombinant protein at a concentration of 200 ng/mL, blasticidin at a concentration of 20 µg/mL and puromycin at a concentration of 2 µg/mL, was placed in the plate and allowed to stand overnight at 37°C.

All of the medium was removed from the plate, and 1 mL of N3 medium containing B18R recombinant protein at a concentration of 200 ng/mL was placed in the plate. This resulted in transfection of the mRNA into the cells (Day 3), similar to the previous days. All of the medium was then removed from the plate, and 1 mL of N3 medium containing B18R recombinant protein at a concentration of 200 ng/mL, blasticidin at a concentration of 20 µg/mL and puromycin at a concentration of 2 µg/mL, was placed in the plate and allowed to stand overnight at 37°C. This procedure was repeated until Day 6.

All of the medium was removed from the plate, and 1 mL of N3 medium containing B18R recombinant protein at a concentration of 200 ng/mL was placed in the plate (Day 7). The cells were then cultured until Day 9.

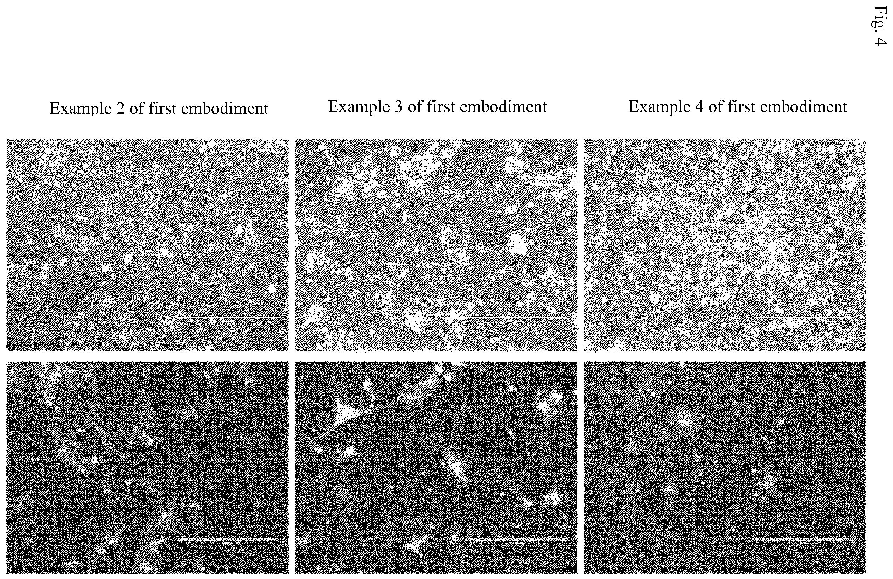

Microscope observation confirmed that neurons had been induced from the iPS cells, as shown in Fig. 4.

### (Example 3 of the first embodiment)

A plate coated with a solubilized basal membrane preparation (Matrigel, Corning) was prepared. After suspending single cell-dispersed iPS cells in 1 mL of human ES/iPS cell-supporting medium (mTeSR1, TEMCELL Technologies) containing B18R recombinant protein at a concentration of 200 ng/mL and ROCK inhibitor, the suspension was added to the plate, seeding the cells, and the suspension was allowed to stand for one day.

The medium was exchanged with 1 mL of xeno-free medium (Pluriton, Stemgent) containing B18R recombinant protein at a concentration of 200 ng/mL and ROCK inhibitor.

A 1.5 mL micro centrifuge tube A and a 1.5 mL micro centrifuge tube B were also prepared.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture.

In tube B there was placed 62.5 µL of low serum medium (Opti-MEM^{R}, Gibco), and then 500 ng of ASCL1-PURO mRNA, 250 ng of DLX2-T2A-BLAST mRNA, 250 ng of MYT1L mRNA and 100 ng of GFP mRNA (Trilink) were added and the mixture was thoroughly agitated to obtain a second reaction mixture. MYT1L is a protein that regulates differentiation to neurons.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, and 1 mL of xeno-free medium (Pluriton, Stemgent) containing B18R recombinant protein at a concentration of 200 ng/mL and ROCK inhibitor was placed in the plate and allowed to stand overnight at 37°C.

This resulted in transfection of the mRNA into the cells (Day 1), similar to the previous day. All of the medium was then removed from the plate, and 1 mL of xeno-free medium (Pluriton, Stemgent) containing B18R recombinant protein at a concentration of 200 ng/mL, blasticidin as an antibiotic at a concentration of 20 µg/mL and puromycin at a concentration of 2 µg/mL and ROCK inhibitor, was placed in the plate and allowed to stand overnight at 37°C.

All of the medium was removed from the plate, and 1 mL of xeno-free medium (Pluriton, Stemgent) containing B18R recombinant protein at a concentration of 200 ng/mL and ROCK inhibitor was placed in the plate. This resulted in transfection of the mRNA into the cells (Day 2), similar to the previous days. All of the medium was then removed from the plate, and 1 mL of N3 medium containing B18R recombinant protein at a concentration of 200 ng/mL, blasticidin at a concentration of 20 µg/mL and puromycin at a concentration of 2 µg/mL, was placed in the plate and allowed to stand overnight at 37°C.

All of the medium was removed from the plate, and 1 mL of N3 medium containing B18R recombinant protein at a concentration of 200 ng/mL was placed in the plate. This resulted in transfection of the mRNA into the cells (Day 3), similar to the previous days. All of the medium was then removed from the plate, and 1 mL of N3 medium containing B18R recombinant protein at a concentration of 200 ng/mL, blasticidin at a concentration of 20 µg/mL and puromycin at a concentration of 2 µg/mL, was placed in the plate and allowed to stand overnight at 37°C. This procedure was repeated until Day 6.

All of the medium was removed from the plate, and 1 mL of N3 medium containing B18R recombinant protein at a concentration of 200 ng/mL was placed in the plate (Day 7). The cells were then cultured until Day 21.

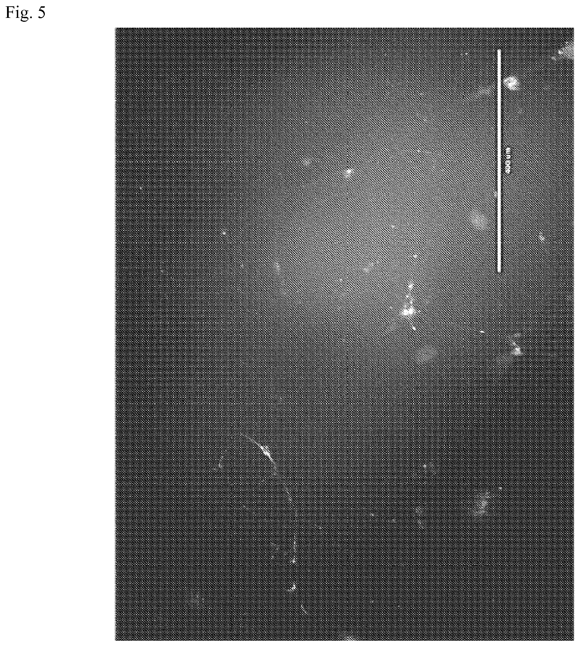

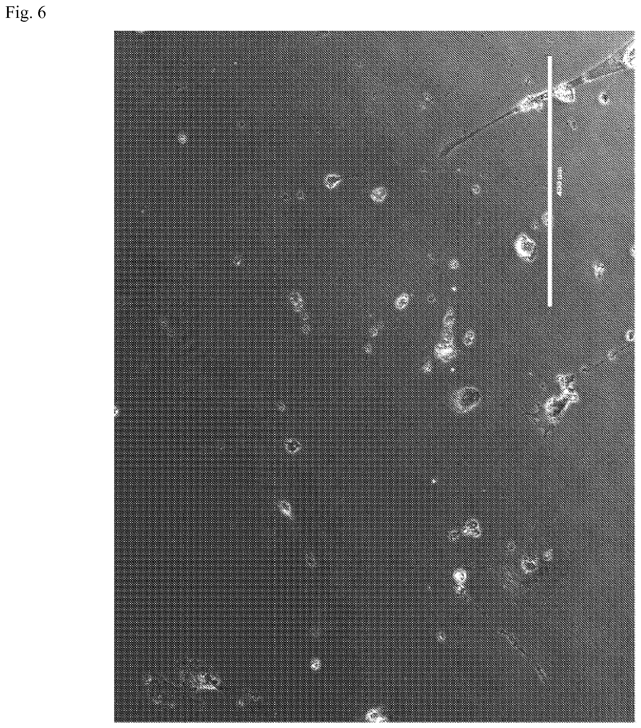

Microscope observation on Day 9 confirmed that neurons had been induced from the iPS cells, as shown in Fig. 4. In addition, when the inhibitory neuron marker GAD67 antibody (MAB5406, Millipore) was used for fluorescent immunostaining of the cells on Day 21, as in Example 1 of the first embodiment, the neurons induced from the iPS cells were confirmed to be expressing the inhibitory neuron marker GAD67, as shown in Fig. 5 and Fig. 6. Moreover, the cells in which the mRNA had not been introduced had been efficiently killed with blasticidin and puromycin, while the cells in which the mRNA had been introduced had been selectively allowed to survived. This also demonstrated that introduction of MYT1L mRNA into cells results in more efficient induction of neurons. Moreover, performing selection with both blasticidin and puromycin reduced the number of transformants that had proliferation potency with incomplete reprogramming.

### (Example 4 of the first embodiment)

A plate coated with a solubilized basal membrane preparation (Matrigel, Corning) was prepared. After suspending single cell-dispersed iPS cells in 1 mL of human ES/iPS cell-supporting medium (mTeSR1, TEMCELL Technologies) containing B18R recombinant protein at a concentration of 200 ng/mL and ROCK inhibitor, the suspension was added to the plate, seeding the cells, and the suspension was allowed to stand for one day.

The medium was exchanged with 1 mL of xeno-free medium (Pluriton, Stemgent) containing B18R recombinant protein at a concentration of 200 ng/mL and ROCK inhibitor.

A 1.5 mL micro centrifuge tube A and a 1.5 mL micro centrifuge tube B were also prepared.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture.

In tube B there was placed 62.5 µL of low serum medium (Opti-MEM^{R}, Gibco), and then 500 ng of ASCL1 mRNA, 250 ng of DLX2-T2A-BLAST mRNA, 250 ng of MYT1L mRNA and 100 ng of GFP mRNA (Trilink) were added and the mixture was thoroughly agitated to obtain a second reaction mixture.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, and 1 mL of xeno-free medium (Pluriton, Stemgent) containing B18R recombinant protein at a concentration of 200 ng/mL and ROCK inhibitor was placed in the plate and allowed to stand overnight at 37°C.

This resulted in transfection of the mRNA into the cells (Day 1), similar to the previous day. All of the medium was then removed from the plate, and 1 mL of xeno-free medium (Pluriton, Stemgent) containing B18R recombinant protein at a concentration of 200 ng/mL and blasticidin as an antibiotic at a concentration of 20 ng/mL and ROCK inhibitor, was placed in the plate and allowed to stand overnight at 37°C.

All of the medium was removed from the plate, and 1 mL of xeno-free medium (Pluriton, Stemgent) containing B18R recombinant protein at a concentration of 200 ng/mL and ROCK inhibitor was placed in the plate. This resulted in transfection of the mRNA into the cells (Day 2), similar to the previous days. All of the medium was then removed from the plate, and 1 mL of N3 medium containing B18R recombinant protein at a concentration of 200 ng/mL and blasticidin at a concentration of 20 ng/mL, was placed in the plate and allowed to stand overnight at 37°C.

All of the medium was removed from the plate, and 1 mL of N3 medium containing B18R recombinant protein at a concentration of 200 ng/mL was placed in the plate. This resulted in transfection of the mRNA into the cells (Day 3), similar to the previous days. All of the medium was then removed from the plate, and 1 mL of N3 medium containing B18R recombinant protein at a concentration of 200 ng/mL and blasticidin at a concentration of 20 ng/mL, was placed in the plate and allowed to stand overnight at 37°C. This procedure was repeated until Day 6.

All of the medium was removed from the plate, and 1 mL of N3 medium containing B18R recombinant protein at a concentration of 200 ng/mL was placed in the plate (Day 7). The cells were then cultured until Day 21.

Microscope observation confirmed that neurons had been induced from the iPS cells, as shown in Fig. 4.

### (Example 5 of the first embodiment)

A plate coated with a solubilized basal membrane preparation (Matrigel, Corning) was prepared. After suspending 2 × 10⁵ single cell-dispersed iPS cells in 1 mL of human ES/iPS cell-supporting medium (mTeSR1, TEMCELL Technologies) containing B18R recombinant protein at a concentration of 200 ng/mL and ROCK inhibitor, the suspension was added to the plate, seeding the cells, and the suspension was allowed to stand for one day.

The medium was exchanged with 1 mL of xeno-free medium (Pluriton, Stemgent) containing B18R recombinant protein at a concentration of 200 ng/mL, ROCK inhibitor, 500 nmol/L of A83-1 as a selective inhibitor of ALK5, ALK4 and ALK7, 200 ng/mL of growth factor SHH (sonic hedgehog), 100 nmol/L of growth factor LDN, 100 ng/mL of growth factor FGF8, 3 µmol/L of the GSK-3β inhibitor CHIR99021, and 2 µmol/L of the differentiation promoting reagent purmorphamine. This medium will be referred to as "Pluri-NPC medium".

Also, a 1.5 mL micro centrifuge tube A and a 1.5 mL micro centrifuge tube B were prepared.

In tube A there was placed 62.6 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture.

In tube B there was placed 62.5 µL of low serum medium (Opti-MEM^{R}, Gibco), and then 200 ng of NGN2-T2A-PURO mRNA, 200 ng of ASCL1-T2A-PURO mRNA, 200 ng of NURR1 mRNA, 200 ng of LMX1A mRNA, 200 ng of EN1 (Engrailed-1) mRNA, 200 ng of PITX3 mRNA, 200 ng of FOXA2 mRNA and 100 ng of GFP mRNA (Trilink) were added and the mixture was thoroughly agitated to obtain a second reaction mixture.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, Pluri-NPC medium was placed in the plate and the mixture was allowed to stand overnight at 37°C.

This resulted in transfection of the mRNA into the cells (Day 1), similar to the previous day. All of the medium was then removed from the plate, Pluri-NPC medium containing puromycin at a concentration of 2 µg/mL was placed in the plate and the mixture was allowed to stand overnight at 37°C.

Next, 1 mL of N3 medium was prepared containing B18R recombinant protein at a concentration of 200 ng/mL, 200 ng/mL of growth factor SHH, 100 nmol/L of growth factor LDN, 100 ng/mL of growth factor FGF8, 3 µmol/L of CHIR and 2 µmol/L of purmorphamine. This medium will be referred to as "N3-C medium". All of the medium was then removed from the plate, and Pluri-NPC medium was placed in the plate. This resulted in transfection of the mRNA into the cells (Day 2), similar to the previous days. All of the medium was then removed from the plate, 1 mL of N3-C medium containing puromycin at a concentration of 2 µg/mL was placed in the plate and the mixture was allowed to stand overnight at 37°C.

All of the medium was then removed from the plate, and fresh N3-C medium was placed in the plate. This resulted in transfection of the mRNA into the cells (Day 3), similar to the previous days. Next, 1 mL of N3-C medium containing puromycin at a concentration of 2 µg/mL was placed in the plate and the mixture was allowed to stand overnight at 37°C. This likewise resulted in transfection of the mRNA into the cells on Day 4 as well.

All of the medium was then removed from the plate, and 1 mL of puromycin-free N3-C medium was placed in the plate (Day 5).

Immunostaining of the cells was carried out on Day 7. Buffer containing rabbit anti-TUJ1 antibody (Covance) at 1:1000 and sheep anti-TH antibody (Pel-Freez Biologicals) at 1:1000 was added to the plate, and the mixture was allowed to stand overnight at 4°C. Next, donkey anti-mouse IgG (H+L) secondary antibody Alexa Fluor^{R} 555 complex (Thermofisher, A-21428) and donkey anti-rabbit IgG (H+L)secondary antibody Alexa Fluor^{R} 647 complex (Thermofisher, A31573) were added to the plate, and the cells were observed under a microscope.

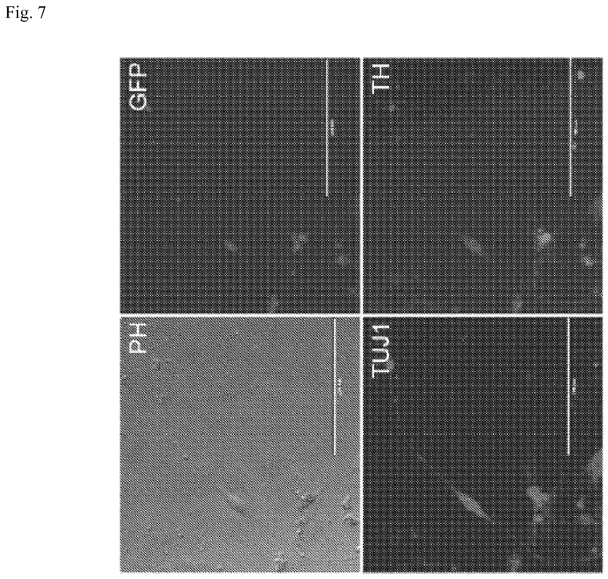

As a result, as shown in Fig. 7, the cells were confirmed to be positive for TH as a specific marker for dopamine-producing neurons and TUJ1 as a general marker for neurons. Virtually no GFP was expressed, however. This indicates that safe dopamine-producing neurons had been obtained, with the exogenous RNA degraded.

### (Example 6 of the first embodiment)

After seeding 2 × 10⁵ iPS cells on a plate in the same manner as Example 5 of the first embodiment, they were allowed to stand for one day. The medium was then exchanged with Pluri-NPC medium. Tube A containing a first reaction mixture was prepared in the same manner as Example 5 of the first embodiment.

In tube B there was placed 62.5 µL of low serum medium (Opti-MEM^{R}, Gibco), and then 200 ng of ASCL1-T2A-PURO mRNA, 200 ng of NURR1 mRNA, 200 ng of LMX1A mRNA, 200 ng of EN1 mRNA, 200 ng of PITX3 mRNA, 200 ng of FOXA2 mRNA and 100 ng of GFP mRNA (Trilink) were added and the mixture was thoroughly agitated to obtain a second reaction mixture.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, Pluri-NPC medium was placed in the plate and the mixture was allowed to stand overnight at 37°C.

This resulted in transfection of the mRNA into the cells (Day 1), similar to the previous day. All of the medium was then removed from the plate, Pluri-NPC medium containing puromycin at a concentration of 2 µg/mL was placed in the plate and the mixture was allowed to stand overnight at 37°C.

All of the medium was then removed from the plate, and Pluri-NPC medium was placed in the plate. This resulted in transfection of the mRNA into the cells (Day 2), similar to the previous days. All of the medium was then removed from the plate, 1 mL of N3-C medium containing puromycin at a concentration of 2 µg/mL was placed in the plate and the mixture was allowed to stand overnight at 37°C.

All of the medium was then removed from the plate, and fresh N3-C medium was placed in the plate. This resulted in transfection of the mRNA into the cells (Day 3), similar to the previous days. Next, 1 mL of N3-C medium containing puromycin at a concentration of 2 µg/mL was placed in the plate and the mixture was allowed to stand overnight at 37°C. This likewise resulted in transfection of the mRNA into the cells on Day 4 as well.

All of the medium was then removed from the plate, and 1 mL of puromycin-free N3-C medium was placed in the plate (Day 5).

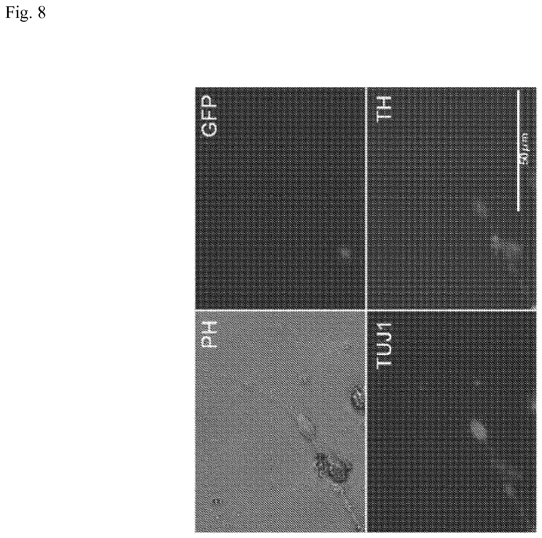

The cells were immunostained on Day 7 in the same manner as Example 5 of the first embodiment. As a result, as shown in Fig. 8, the cells were confirmed to be positive for TH and TUJ1. Virtually no GFP was expressed, however.

### (Example 7 of the first embodiment)

iPS cells were seeded on a plate and allowed to stand for one day in the same manner as Example 5 of the first embodiment, except that the number of iPS cells was 4 × 10⁵. The medium was then exchanged with Pluri-NPC medium. Tube A containing a first reaction mixture was prepared in the same manner as Example 5 of the first embodiment.

In tube B there was placed 62.5 µL of low serum medium (Opti-MEM^{R}, Gibco), and then 500 ng of ASCL1-T2A-PURO mRNA, 200 ng of NURR1 mRNA, 200 ng of LMX1A mRNA, 200 ng of EN1 mRNA, 200 ng of PITX3 mRNA, 200 ng of FOXA2 mRNA and 100 ng of GFP mRNA (Trilink) were added and the mixture was thoroughly agitated to obtain a second reaction mixture.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, Pluri-NPC medium was placed in the plate and the mixture was allowed to stand overnight at 37°C.

All of the medium was removed from the plate, and the cells were transfected with the mRNA in the same manner as the previous day (Day 1). All of the medium was then removed from the plate, Pluri-NPC medium containing puromycin at a concentration of 2 µg/mL was placed in the plate and the mixture was allowed to stand overnight at 37°C.

All of the medium was then removed from the plate, and Pluri-NPC medium was placed in the plate. This resulted in transfection of the mRNA into the cells (Day 2), similar to the previous days. All of the medium was then removed from the plate, 1 mL of N3-C medium containing puromycin at a concentration of 2 µg/mL was placed in the plate and the mixture was allowed to stand overnight at 37°C.

All of the medium was then removed from the plate, and fresh N3-C medium was placed in the plate. This resulted in transfection of the mRNA into the cells (Day 3), similar to the previous days. Next, 1 mL of N3-C medium containing puromycin at a concentration of 2 µg/mL was placed in the plate and the mixture was allowed to stand overnight at 37°C. This likewise resulted in transfection of the mRNA into the cells on Day 4 and Day 5 as well.

All of the medium was then removed from the plate, and 1 mL of puromycin-free N3-C medium was placed in the plate (Day 6).

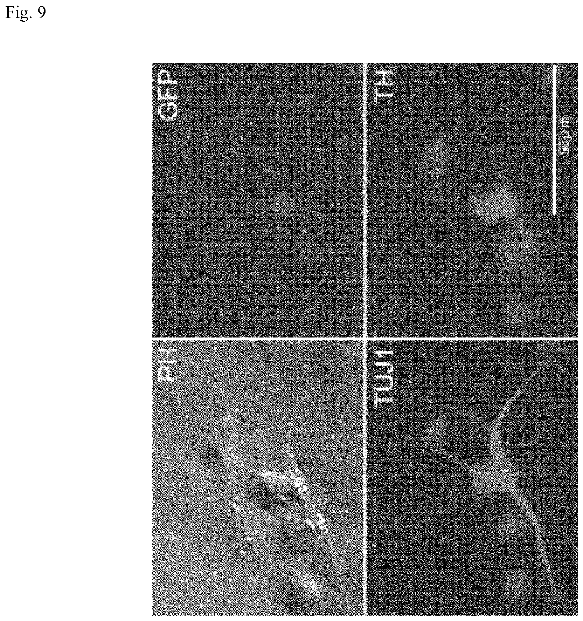

The cells were immunostained on Day 7 in the same manner as Example 5 of the first embodiment. As a result, as shown in Fig. 9, the cells were confirmed to be positive for TH and TUJ1. Virtually no GFP was expressed, however.

### (Example 8 of the first embodiment)

iPS cells were seeded on a plate and allowed to stand for one day in the same manner as Example 5 of the first embodiment, except that the number of iPS cells was 2 × 10⁵ or 4 × 10⁵. The medium was then exchanged with Pluri-NPC medium. Tube A containing a first reaction mixture was prepared in the same manner as Example 5 of the first embodiment.

In tube B there was placed 62.5 µL of low serum medium (Opti-MEM^{R}, Gibco), and then 500 ng of NGN2-T2A-PURO mRNA and 100 ng of GFP mRNA (Trilink) were added and the mixture was thoroughly agitated to obtain a second reaction mixture.

The second reaction mixture was added to the first reaction mixture in tube A to obtain a mixed reaction solution, and then tube A was lightly tapped for 10 minutes at room temperature to form liposomes. The mixed reaction solution was then added to the plate and allowed to stand at 37°C for 6 to 8 hours. This resulted in transfection of the mRNA into the cells (Day 0). All of the medium was then removed from the plate, Pluri-NPC medium was placed in the plate and the mixture was allowed to stand overnight at 37°C.

This resulted in transfection of the mRNA into the cells (Day 1), similar to the previous day. All of the medium was then removed from the plate, Pluri-NPC medium containing puromycin at a concentration of 2 µg/mL was placed in the plate and the mixture was allowed to stand overnight at 37°C.

All of the medium was then removed from the plate, and Pluri-NPC medium was placed in the plate. The cells were then transfected with 200 ng of NGN2-T2A-PURO mRNA, 200 ng of ASCL1-T2A-PURO mRNA, 200 ng of NURR1 mRNA, 200 ng of LMX1A mRNA, 200 ng of EN1 mRNA, 200 ng of PITX3 mRNA, 200 ng of FOXA2 and 100 ng of GFP mRNA (Day 2). All of the medium was removed from the plate, 1 mL of N3-C medium containing puromycin at a concentration of 2 µg/mL was placed in the plate and the mixture was allowed to stand overnight at 37°C.

All of the medium was then removed from the plate, and fresh N3-C medium was placed in the plate. This resulted in transfection of the mRNA into the cells (Day 3), similar to the previous days. Next, 1 mL of N3-C medium containing puromycin at a concentration of 2 µg/mL was placed in the plate and the mixture was allowed to stand overnight at 37°C. This likewise resulted in transfection of the mRNA into the cells on Day 4 as well.

All of the medium was then removed from the plate, and fresh N3-C medium was placed in the plate. This resulted in transfection of the mRNA into the cells (Day 5), similar to the previous days. After placing 1 mL of N3-C medium containing puromycin at a concentration of 2 µg/mL and B18R recombinant protein (eBioscience) into the plate, it was allowed to stand overnight at 37°C.

All of the medium was then removed from the plate, and 1 mL of puromycin-free N3-C medium was placed in the plate (Day 6).

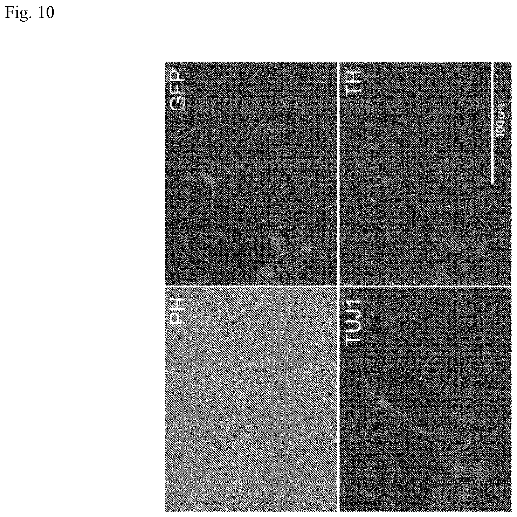

The cells were immunostained on Day 7 in the same manner as Example 5 of the first embodiment. As a result, as shown in Fig. 10, the cells were confirmed to be positive for TH and TUJ1. Virtually no GFP was expressed, however. This indicates that safe dopamine-producing neurons had been obtained, with the exogenous RNA degraded.

### (Comparative Example for the first embodiment)

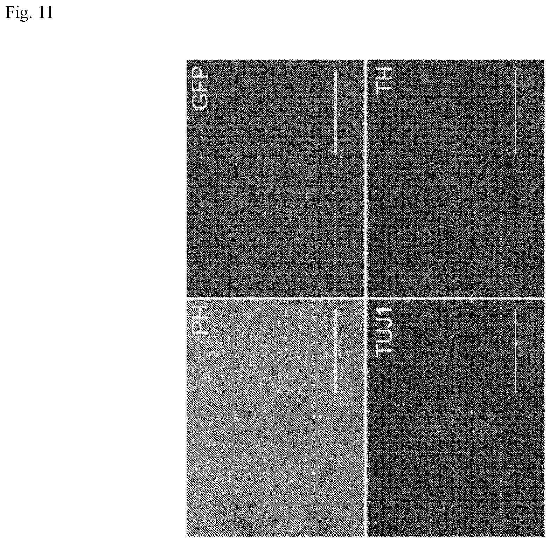

Pluri-NPC medium and N3-C medium are media used when inducing dopamine-producing neurons from iPS cells using only hormones. For the Comparative Example, cells were cultured with exchange of medium in the same manner as Examples 5 to 8 of the first embodiment, except that inducing factor RNA was not introduced into the iPS cells, and the cells were immunostained on Day 7. As a result, as shown in Fig. 11, the cells were confirmed to be negative for TH and TUJ1. The morphology also differed from the morphology of neurons.

### (Second embodiment)

The nerve cell production method according to the second embodiment includes preparing cells, introducing reprogramming factor RNA into the cells, and introducing inducing factor RNA into the cells in which the reprogramming factor RNA has been introduced, to cause their differentiation into nerve cells.

In the nerve cell production method of the second embodiment, reprogramming factor RNA may be introduced into cells and inducing factor RNA may be introduced into the reprogramming factor RNA-introduced cells, all in the same culturing vessel. For example, after the reprogramming factor RNA has been introduced into the cells, the inducing factor RNA may be introduced into the reprogramming factor RNA-introduced cells without detaching the reprogramming factor RNA-introduced cells from the culturing vessel. The inducing factor RNA may also be introduced into the cells on the day following introduction of the reprogramming factor RNA into the cells.

Alternatively, in the nerve cell production method of the second embodiment, after the reprogramming factor RNA has been introduced into the cells, the inducing factor RNA may be introduced into the reprogramming factor RNA-introduced cells after detaching the reprogramming factor RNA-introduced cells from the culturing vessel and seeding the reprogramming factor RNA-introduced cells into a different culturing vessel.

Examples of cells into which the reprogramming factor is to be introduced include differentiated cells (somatic cells) such as fibroblasts, blood cells, dental pulp stem cells, keratinocytes, hair papilla cells, oral epithelial cells and somatic stem progenitor cells.

Blood cells are separated from blood. The blood may be, but is not limited to, peripheral blood and umbilical cord blood. The blood may be harvested from an adult or from a juvenile. An anticoagulant such as ethylenediaminetetraacetic acid (EDTA), heparin or biologically standardized blood storage Solution A (ACD-A) may be used for blood harvesting.

Blood cells are, for example, nucleated cells such as monocytes, neutrophils, eosinophils, basophils and lymphocytes, including no erythrocytes, granulocytes or platelets. The blood cells may be vascular endothelial precursor cells, blood stem cells or progenitor cells, T cells or B cells. T cells may be αβ T cells, for example.

Monocytes are separated from blood using a blood cell separation medium and a centrifugal separation apparatus. The method for separating monocytes when using Ficoll (GE Healthcare) as the blood cell separation medium is as follows.

Because the separation precision for monocytes tends to be poor at low temperature, the centrifuge is set to between 4°C and 42°C, and preferably 18°C. After collecting 10 µL to 50 mL of blood from an adult or juvenile human, a chelating agent containing EDTA is added and thoroughly mixed with the blood to prevent solidification of the blood. Also, medium for human lymphocyte separation Ficoll-Paque PREMIUM, GE Healthcare, Japan) is dispensed into two 15 mL tubes at 5 mL each. After adding 5 mL of PBS to 5 mL of the blood for dilution, 5 mL of each is overlaid onto the human lymphocyte separation medium in the tubes. During this time, the diluted blood is slowly added onto the medium while causing it to slide on the tube wall so as not to disturb the interface.

The solution in the tube is centrifuged at between 10 × g and 1000 × g, and preferably 400 × g, for between 5 minutes and 2 hours, and preferably 30 minutes, at between 4°C and 42°C, and preferably 18°C. After centrifugation, a white cloudy intermediate layer appears in the tube. The white cloudy intermediate layer includes monocytes. The white cloudy intermediate layer in each tube is slowly collected with a Pipetman and transferred to a new 15 mL tube. The lower layer is not handled during this time. Approximately 1 mL of the white cloudy intermediate layer can be collected from each tube. The intermediate layers of two tubes are combined and transferred to a single tube.

After adding between 1 mL and 48 mL, and preferably 12 mL of PBS to the collected monocytes, the solution is further centrifuged at between 10 × g and 1000 × g, and preferably 200 × g, at between 4°C and 42°C, and preferably 18°C, for between 1 minute and 60 minutes, and preferably 10 minutes. Next, an aspirator is used to draw out and remove the supernatant of the solution, and between 1 mL and 12 mL, and preferably 3 mL, of a serum-free hematopoietic cell medium of known composition (X-VIVO^{R} 10, Lonza) is added to obtain a monocyte suspension. A 10 µL portion of the monocyte suspension is stained with Trypan blue and the count is determined with a hemocytometer.

The method for separating the monocytes when using a Vacutainer^{R} (BD) as the blood sampling tube is as follows.

Because the separation precision for monocytes tends to be poor at low temperature, the centrifuge is set to between 4°C and 42°C, and preferably 18°C. A blood sampling tube (Vacutainer^{R}, BD) is used to harvest 8 mL of blood from an adult or juvenile human, and inverting mixing is carried out for mixture with an anticoagulant. The balance is then adjusted, and the solution is centrifuged with a swing rotor at between 4°C and 42°C, and preferably 18°C, at between 100 × g and 3000 × g, and preferably between 1500 × g and 1800 × g, for between 1 minute and 60 minutes, and preferably 20 minutes. After centrifugation, the upper layer (blood plasma layer) is removed, and pipetting is performed to obtain the mononuclear cell layer and a suspension in which the gel-adhering blood cells are suspended. The obtained suspension is transformed to a separate 15 mL tube.

After adding between 1 mL and 14 mL, and preferably 12 mL of PBS to the suspension in the 15 mL tube, the suspension is centrifuged at between 4°C and 42°C, and preferably 18°C, at between 100 × g and 3000 × g, and preferably 200 × g, for between 1 minute and 60 minutes, and preferably 5 minutes. After centrifugation, the supernatant is removed with an aspirator. A hemolytic agent (PharmLyse^{R}, 10-fold concentration, BD) is diluted to 1-fold concentration with sterilized water. The pellet in the 15 mL tube is broken up by tapping, and between 1 mL and 14 mL, and preferably 1 mL, of hemolytic agent is added. It is then shielded from light at room temperature, and the solution is allowed to stand for between 1 minute and 60 minutes, and preferably 1 minute.

After then adding between 1 mL and 14 mL, and preferably 12 mL of PBS to the 15 mL tube, it is centrifuged at between 4°C and 42°C, and preferably room temperature, at between 100 × g and 3000 × g, and preferably 200 × g, for between 1 minute and 60 minutes, and preferably 5 minutes. After centrifugation, an aspirator is used to remove the supernatant, and between 1 mL and 15 mL, and preferably 3 mL, of a serum-free hematopoietic cell medium of known composition (X-VIVO^{R} 10, Lonza) is added to obtain a monocyte suspension. A 10 µL portion of the monocyte suspension is stained with Trypan blue and the count is determined with a hemocytometer.

The method for separating the monocytes from blood is not limited to the method described above, and it may be separation of the monocytes from the blood using a dialysis membrane, for example. A filter, such as a whole blood monocyte concentration Purecell Select System^{R} (PALL), a blood cell removing purifier (Cellsorba E^{R}, Asahi Kasei Corp.) or a platelet preparation leukocyte removal filter (SEPACELL PL^{R}, PLX-5B-SCD, Asahi Kasei Corp.) may also be used.

The monocytes may be separated using an erythrocyte separating agent that is able to separate nucleated cells by gravity settling or centrifugal separation of erythrocytes. Examples of erythrocyte separating agents include HetaSep^{R} (Stemcell Technologies) and HES40 (Nipro).

The monocytes used may be CTL-UP1, marketed by Cellular Technology Limited, or PBMC-001 by Sanguine Biosciences.

Alternatively, the blood cells may be blood cells that have been cryopreserved using a cell cryopreservation liquid such as CELLBANKER 1, STEMCELLBANKER GMP grade, or STEMCELLBANKER DMSO-free GMP grade (Zenoaq), and then thawed.

For thawing of the monocytes, first between 1 mL and 15 mL, and preferably 8 mL of serum-free hematopoietic cell medium of known composition (X-VIVO^{R} 10, Lonza) is placed in a 15 mL tube, and the tube containing the frozen monocytes is set in a hot bath at from 4°C to 42°C and preferably 37°C, to dissolve the monocytes. Next, while some of the ice is remaining, the tube containing the monocytes is pulled out from the hot bath and transferred to a tube containing serum-free hematopoietic cell medium of known composition. A 10 µL portion of the monocyte suspension is stained with Trypan blue and the count is determined with a hemocytometer.

The blood cells may be separated based on the presence of a cell surface marker. Blood stem cells and progenitor cells are CD34-positive. T cells are positive for CD3, CD4 or CD8. B cells are positive for CD10, CD19 or CD20. Blood stem cells or progenitor cells, T cells, or B cells are separated from blood cells using an automatic magnetic cell separator and immunomagnetic beads, for example. Alternatively, pre-separated monocytes may be prepared. However, the blood cells that have not been separated based on the presence of a cell surface marker may also be used.

CD34-positive cells are stem cells or stem cell progenitors, and tend to be easily reprogrammable. When iPS cells are prepared using T cells, which are CD3-positive cells, the T cell-derived iPS cells retain their TCR recombination form, so that it is generally possible to efficiently induce differentiation to T cells.

The method for separating CD34-positive cells is as follows.

There is additionally prepared a blood cell culture medium (blood stem cell or progenitor cell medium) by adding 10 µL of IL-6 (100 µg/mL), 10 µL of SCF (300 µg/mL), 10 µL of TPO (300 µg/mL), 10 µL of FLT3 ligand (300 µg/mL) and 10 µL of IL-3 (10 µg/mL) to 10 mL of serum-free medium (StemSpan H3000, Stemcell Technologies).

The blood cell medium is placed in each well of a 6-well plate, to between 1 mL and 6 mL, and preferably 2 mL. In order to prevent evaporation of the medium, between 1 mL and 6 mL, or 2 mL, of PBS is placed in each of 5 more wells. The 6-well plate is then placed in an incubator at between 4°C and 42°C, and preferably 37°C, and incubated.

A column buffer is prepared with between 10 µL and 1 mL, and preferably 80 µL of EDTA (500 mmol/L) and between 10 µL and 1 mL, and preferably 200 µL of FBS, added to 20 mL of PBS. A monocyte suspension containing between 1 × 10⁴ and 1 × 10⁹, and preferably 2 × 10⁷ monocytes is dispensed into a 15 mL tube, and the monocyte suspension is centrifuged for 10 minutes at between 4°C and 42°C, and preferably 4°C, at between 100 × g and 3000 × g, and preferably 300 × g. After centrifugation, the supernatant is removed and the monocytes are suspended in between 100 µL and 1 mL, and preferably 300 µL, of column buffer.

Next, between 10 µL and 1 mL, and preferably 100 µL, of FcR blocking reagent (Miltenyi Biotec) and between 10 µL and 1 mL, and preferably 100 µL, of a CD34 microbeads kit (Miltenyi Biotec) are added to the monocyte suspension in the 15 mL tube. FcR blocking reagent is used to increase the microbeads-labeling specificity. The monocyte suspension is then mixed in, and the mixture is allowed to stand at between 4°C and 42°C, and preferably 4°C, for between 1 minute and 2 hours, and preferably 30 minutes.

Next, between 1 mL and 15 mL, and preferably 10 mL, of column buffer is added to the monocyte suspension in the 15 mL tube for dilution, and the mixture is centrifuged at between 4°C and 42°C, and preferably 4°C, at between 100 × g and 1000 × g, and preferably 300 × g, for between 1 minute and 2 hours, and preferably 10 minutes. After centrifugation, the supernatant in the 15 mL tube is removed with an aspirator, and between 10 µL and 10 mL, and preferably 500 µL, of column buffer is added for resuspension.

An automatic magnetic cell separator column (MS column, Miltenyi Biotec) is mounted in an automatic magnetic cell separator (MiniMACS Separation Unit, Miltenyi Biotec), and between 10 µL and 10 mL, and preferably 500 µL, of column buffer is placed in the column and rinsing is carried out. The monocytes are then placed in the column. After then placing between 10 µL and 10 mL, and preferably 500 µL of column buffer in the column, the column is rinsed from 1 to 10 times, and preferably 3 times. The column is then removed from the automatic magnetic cell separator and placed in a 15 mL tube. Next, between 10 µL and 10 mL, and preferably 1000 µL, of column buffer is placed in the column and a syringe is rapidly pressed to discharge the CD34-positive cells into the 15 mL tube.

A 10 µL portion of the CD34-positive cell suspension is dyed with Trypan blue, and the cell count is determined using a blood cell counting chamber. The CD34-positive cell suspension in the 15 mL tube is centrifuged at between 4°C and 42°C, and preferably 4°C, at between 100 × g and 1000 × g, and preferably 300 × g, for between 1 minute and 2 hours, and preferably 10 minutes. After centrifugation, the supernatant is removed with an aspirator. The CD34-positive cells are resuspended in preheated blood cell medium, and the CD34-positive cells are spread onto a culture plate. The CD34-positive cells are then cultured for 6 days at between 4°C and 42°C, and preferably 37°C, with between 1% and 20%, and preferably 5% CO₂. There is no need for medium exchange during this procedure.

The method for isolating cells with a marker other than CD34 is the same as the method for isolating CD34-positive cells.

The reprogramming factor RNA to be introduced into the cells includes OCT3/4 mRNA, SOX2 mRNA, KLF4 mRNA, and c-MYC mRNA, for example. The reprogramming factor RNA used may be OCT3/4-modified M₃O. The reprogramming factor RNA may further include mRNA of at least one factor selected from the group consisting of LIN28A, LIN28B, GLIS1, p53-dominant negative, p53-P275S, L-MYC, NANOG, DPPA2, DPPA4, DPPA5, ZIC3, BCL-2, E-RAS, TPT1, SALL2, NAC1, DAX1, TERT, ZNF206, FOXD3, REX1, UTF1, KLF2, KLF5, ESRRB, miR-291-3p, miR-294, miR-295, NR5A1, NR5A2, TBX3, MBD3sh, TH2A and TH2B. These mRNAs are available from TriLink.

The mRNA in the inducing factor RNA may be modified with one or more selected from the group consisting of pseudouridine (Ψ), 5-methyluridine (5meU), N1-methylpseudouridine (me1Ψ), 5-methoxyuridine (5moU), 5-hydroxymethyluridine (5hmU), 5-formyluridine (5fU), 5-carboxymethyl ester uridine (5camU), thienoguanosine (thG), N4-methylcytidine (me4C), 5-methylcytidine (m5C), 5-methoxycytidine (5 moC), 5-hydroxymethylcytidine (5hmC), 5-hydroxycytidine (5hoC), 5-formylcytidine (5fC), 5-carboxycytidine (5caC), N6-methyl-2-aminoadenosine m6DAP), diaminopurine (DAP), 5-methyluridine (m5U), 2'-O-methyluridine (Um or m2'-OU), 2-thiouridine (s2U) and N6-methyladenosine (m6A).

The mRNA may also be polyadenylated.

The mRNA may be prepared by polyadenylation of (IVT)RNA that is transcribed *in vitro.* The mRNA may also be polyadenylated during IVT, using a DNA template coding for poly(A) ends. The mRNA may also be capped. Most of the mRNA molecules are preferably given caps to maximize expression efficiency in the cells. The mRNA may also have a 5'cap[m7G(5')ppp(5')G] structure. This sequence stabilizes mRNA and promotes transcription. In the case of mRNA containing 5'-triphosphate, the 5'-triphosphate may be removed by dephosphorylation treatment. The mRNA may also have [3'O-Me-m7G(5')ppp(5')G] as an Anti-Reverse Cap Analog (ARCA). ARCA is a sequence inserted before the transcription initiation site, and it doubles the efficiency of the mRNA to be transcribed. The mRNA may also have a PolyA tail.

The mRNA may also be replicative RNA with the ability to self-replicate. Replicative RNA is RNA with the ability to self-replicate, and it differs from ordinary RNA in that it has the ability to express proteins necessary for replication of RNA. Replicative RNA is derived from Venezuelan Equine Encephalitis (VEE) virus, a type of alpha virus. Transfecting cells with replicative RNA allows the cells to express RNA that will continue to produce the reprogramming factor, thus making it possible to eliminate repeated introduction of reprogramming factor RNA into the cells.

The replicative RNA sequence may include sequences obtained from alpha viruses selected from the group consisting of alpha virus replicon RNA, Eastern Equine Encephalitis virus (EEE), Venezuelan Equine Encephalitis virus (VEE), Everglades virus, Mucambo virus, Pixuna virus and Western Equine Encephalitis virus (WEE).

The replicative RNA may also include sequences obtained from alpha viruses selected from the group consisting of Sindbis virus, Semliki Forest virus, Middelburg virus, Chikungunya virus, O'nyong-nyong virus, Ross River virus, Barmah Forest virus, Getah virus, Sagiyama virus, Bebaru virus, Mayaro virus, Una virus, Aura virus, Whataroa virus, Babanki virus, Kyzylagach virus, Highlands J virus, Fort Morgan virus, Ndumu virus and Buggy Creek virus.

The replicative RNA includes, from the 5' end to the 3' end, (VEE RNA replicase)-(promoter)-(RFl)-(self-cleaving peptide)-(RF2)-(self-cleaving peptide)-(RF3)-(IRES or core promoter)-(RF4)-(IRES or arbitrary promoter)-(arbitrary selectable marker)-(VEE 3'UTR and polyA tail)-(arbitrary selectable marker)-promoter, for example. The RF1-4 mentioned above is a factor that induces dedifferentiation of cells to pluripotent cells. The RF2-3, RF3-4 and RF4 mentioned above are optional. The RF1-4 may be selected from among the group consisting of OCT3/4, KLF4, SOX-2, c-MYC, LIN28A, LIN28B, GLIS1, FOXH1, p53-dominant negative, p53-P275S, L-MYC, NANOG, DPPA2, DPPA4, DPPA5, ZIC3, BCL-2, E-RAS, TPT1, SALL2, NAC1, DAX1, TERT, ZNF206, FOXD3, REX1, UTF1, KLF2, KLF5, ESRRB, miR-291-3p, miR-294, miR-295, NR5A1, NR5A2, TBX3, MBD3sh, TH2A and TH2B.

The medium used for culturing of the cells in which the reprogramming factor RNA is to be introduced may be, for example, human ES/iPS medium such as stem cell medium such as PluriQ (MTI-GlobalStem) or Primate ES Cell Medium (ReproCELL).

The stem cell culture medium is not limited to this, however, and various stem cell culture media may be used. For example, Primate ES Cell Medium, Reprostem, ReproFF, ReproFF2, ReproXF (Reprocell), mTeSR1, TeSR2, TeSRE8, ReproTeSR (STEMCELL Technologies), PluriSTEM^{R} Human ES/iPS Medium (Merck), NutriStem^{R} XF/FF Culture Medium for Human iPS and ES Cells, Pluriton reprogramming medium (Stemgent), PluriSTEM^{R}, Stemfit AK02N, Stemfit AK03 (Ajinomoto), ESC-Sure^{R} serum and feeder free medium for hESC/iPS (Applied StemCell), and L7^{R} hPSC Culture System (LONZA) may be used. The stem cell medium is accommodated in a dish, well or tube, for example.

The reprogramming factor RNA is introduced into the cells using an RNA transfection reagent. The RNA transfection reagent used may be mRNA-In^{R} (Molecular Transfer, Inc.).

Alternatively, Lipofectamine MessengerMAX^{R}, for example, may be used as a lipofection reagent for transfection of the reprogramming factor RNA. In addition, the RNA lipofection reagent used may be Lipofectamin^{R} RNAiMAX (Thermo Fisher Scientific), Lipofectamin^{R} 2000, Lipofectamin^{R} 3000, NeonTransfection System (Thermo Fisher Scientific), Stemfect RNA transfection reagent (Stemfect), NextFect^{R} RNA Transfection Reagent (BioScientific), Amaxa^{R} Human T cell Nucleofector^{R} kit (Lonza, VAPA-1002), Amaxa^{R} Human CD34 cell Nucleofector^{R} kit (Lonza, VAPA-1003), or ReproRNA^{R} transfection reagent STEMCELL Technologies).

The reprogramming factor RNA may be introduced into the cells several times. Introduction of the reprogramming factor RNA into the cells is carried out, for example, once every 2 days, or once a day, or repeatedly during a period of from 5 days to 15 days, from 7 days to 13 days, or for 10 days. When the mRNA is replicative RNA, however, introduction of the reprogramming factor RNA into the cells may be only once.

Inducing factor RNA is then introduced into the cells in which the reprogramming factor RNA has been introduced, by the same method as described for the first embodiment.

With the method of the second embodiment it is possible to produce nerve cells from differentiated cells in a short period of time.

### (Example 1 of the second embodiment)

In each well of a well plate there was added 1.5 mL of PBS and a diluted basal membrane matrix solution diluted to an iMatrix-511 (Nippi) concentration of 0.5 µg/cm². The well plate was then placed in an incubator at 37°C for 1 hour or longer. The basal membrane matrix dilute solution was then removed out from each of the wells of the well plate, fibroblasts suspended in 10% FBS medium were seeded at approximately 1 × 10⁵ into each well of the well plate, and the fibroblasts were adhesion cultured.

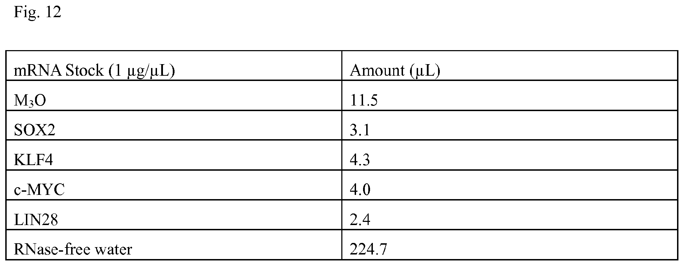

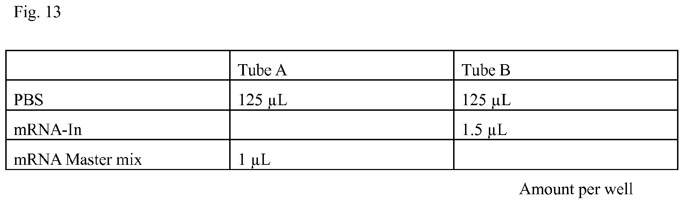

A reprogramming factor mRNA master mix as shown in Fig. 12 was prepared. The medium in each well of the well plate was exchanged with 2 mL of PluriQ^{R} (MTI-GlobalStem). A tube A and a tube B containing the contents listed in Fig. 13 were also prepared. The contents of tube A and tube B were mixed, and the RNA transfection reagent mRNA-In^{R} (Molecular Transfer, Inc.) and the mRNA master mix were combined to form a mixture which was allowed to stand for 10 minutes. The mixture of the RNA-In and mRNA master mix was then added to each well, and the well plate was shaken to disperse the mixture of the RNA-In and mRNA master mix in the medium. The fibroblasts were incubated overnight at 37°C, 5% CO₂ to transfect the cells with the reprogramming factor RNA (Day 0).

The cells were continuously transfected with the reprogramming factor RNA for 9 days thereafter, in the same manner. The cells were thus transfected with the reprogramming factor RNA a total of 10 times.

A transfection medium was prepared by mixing 1.25 mL of xeno-free medium (Pluriton, Stemgent), 0.5 µL of Pluriton Supplement (Stemgent) and 2 µL of 100 ng/µL B18R recombinant protein-containing solution (eBioscience). Before transfection of the inducing factor RNA, the medium in each well was exchanged with transfection medium, and the cells transfected with the reprogramming factor RNA were cultured at 37°C for 2 hours.

NGN2-T2A-PURO mRNA (Trilink) as inducing factor RNA and green fluorescent protein (GFP) mRNA (Trilink) were prepared. The mRNA was capped with Anti-Reverse Cap Analog (ARCA) and polyadenylated, and substituted with 5-methylcytidine and pseudouridine. The mRNA was also purified with a silica membrane, and prepared as a solution in a solvent of 1 mmol/L sodium citrate at pH 6, together with mRNA-introducing reagent (Lipofectamine MessengerMax^{R}, Invitrogen). A 1.5 mL micro centrifuge tube A and a 1.5 mL micro centrifuge tube B were also prepared to match the number of wells.

In tube A there was placed 62.5 µL of low serum medium (Opti-MEM^{R}, Gibco), and then 1.875 µL of mRNA-introducing reagent (Lipofectamine MessengerMax^{R}, Invitrogen) was added and the mixture was thoroughly agitated to obtain a first reaction mixture. Tube A was then lightly tapped for 10 minutes at room temperature, to mix the first reaction mixture.

In tube B there was placed 62.5 µL of low serum medium (Opti-MEM^{R}, Gibco), and then 500 ng of NGN2-T2A-PURO mRNA (Trilink) and 100 ng of GFP mRNA (Trilink) were added and the mixture was thoroughly agitated to obtain a second reaction mixture.

The second reaction mixture was added to the first reaction mixture in tube A to obtain a mixed reaction solution, and then tube A was lightly tapped for 5 minutes at room temperature to form liposomes. The mixed reaction solution was added to each of the wells and allowed to stand at 37°C for 6 to 8 hours. Thus, 500 ng of NGN2 mRNA and 100 ng of GFP mRNA had been added to each well, and the inducing factor RNA had been introduced into the cells that had been transfected with the reprogramming factor RNA (Day 10). The day on which the cells were first transfected with the inducing factor RNA was the day after the final day of transfection of the cells with the reprogramming factor RNA. Next, all of the medium was removed from each well, and xeno-free medium (Pluriton, Stemgent) containing B18R recombinant protein at a concentration of 200 ng/mL was placed in each well and allowed to stand overnight at 37°C.

All of the medium was then removed from each well, and xeno-free medium (Pluriton, Stemgent) containing B18R recombinant protein at a concentration of 200 ng/mL was placed in each well. This resulted in transfection of the inducing factor mRNA into the cells (Day 11), similar to the previous day. Next, all of the medium was then removed from the plate, and xeno-free medium (Pluriton, Stemgent) containing B18R recombinant protein at a concentration of 200 ng/mL and puromycin at a concentration of 2 µg/mL was placed in each well and allowed to stand overnight at 37°C.

All of the medium was then removed from each well, and xeno-free medium (Pluriton, Stemgent) containing B18R recombinant protein at a concentration of 200 ng/mL was placed in each well. This resulted in transfection of the inducing factor mRNA into the cells (Day 12), similar to the previous day. Next, all of the medium was then removed from the plate, and N3 medium containing B18R recombinant protein at a concentration of 200 ng/mL and puromycin at a concentration of 2 µg/mL was placed in each well and allowed to stand overnight at 37°C.

All of the medium was removed from each well, and N3 medium containing B18R recombinant protein at a concentration of 200 ng/mL was placed in each well. This resulted in transfection of the inducing factor mRNA into the cells (Day 13), similar to the previous day. Next, all of the medium was then removed from the plate, and N3 medium containing B18R recombinant protein at a concentration of 200 ng/mL and puromycin at a concentration of 2 µg/mL was placed in each well and allowed to stand overnight at 37°C.

All of the medium was then removed from the plate, and N3 medium containing B18R recombinant protein at a concentration of 200 ng/mL and puromycin at a concentration of 2 µg/mL was placed in each well and allowed to stand overnight at 37°C (Day 14).

All of the medium was removed from the plate, and N3 medium containing B18R recombinant protein at a concentration of 200 ng/mL, and containing no puromycin, was placed in each well and the cells were cultured at 37°C up to Day 20.

The medium was then removed from the plate and the cells were rinsed with PBS. Next, 4% PFA was placed in the plate, and reaction was conducted for 15 minutes at 4°C to fix the cells. The cells were further rinsed twice with PBS, and then the primary antibody was diluted with PBS medium containing 5% CCS and 0.1% Triton and added to the plate. The primary antibodies used were mouse monoclonal antibody (Sigma) for the neuron marker MAP2, and rabbit polyclonal antibody (Synaptic Systems) for the excitatory neuron marker vGLUT.

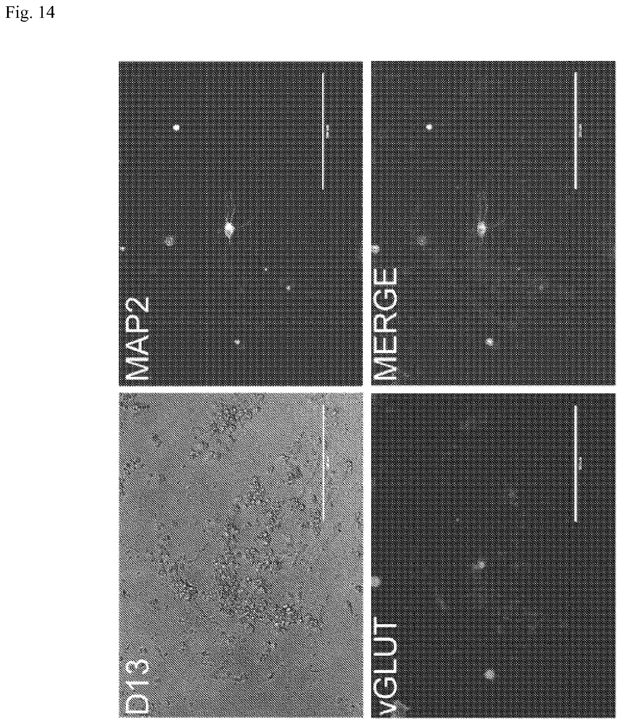

After one hour of reaction at room temperature, PBS was added to the plate and thoroughly mixed with it, and then the PBS was discarded. PBS was again added and discarded, a solution containing fluorescent-labeled donkey anti-mouse IgG (H+L) secondary antibody (Alexa Fluor^{R}, 555, Conjugate, ThermoFisher) and rabbit anti-mouse IgG (H+L) secondary antibody (Alexa Fluor^{R} 647, conjugate, ThermoFisher) was added to the plate, and reaction was conducted at room temperature for 30 minutes. The cells were then rinsed twice with PBS and observed under a fluorescent microscope. As a result, as shown in Fig. 14, the induced neurons were confirmed to be expressing the neuron marker MAP2 and the excitatory neuron marker vGLUT.

### (Example 2 of the second embodiment)

Fibroblasts were prepared in the same manner as Example 1 of the second embodiment, and the cells were transfected with reprogramming factor RNA a total of 10 times. For 5 days thereafter, the cells were cultured with PluriQ^{R} (MTI-GlobalStem) containing TGF-β at a concentration of 2 ng/mL.

The cells were detached from the well plate and suspended in xeno-free medium (Pluriton, Stemgent), and the cells were then reseeded in each well of a fresh well plate. The cells were subsequently transfected with inducing factor RNA in the same manner as Example 1 of the second embodiment.

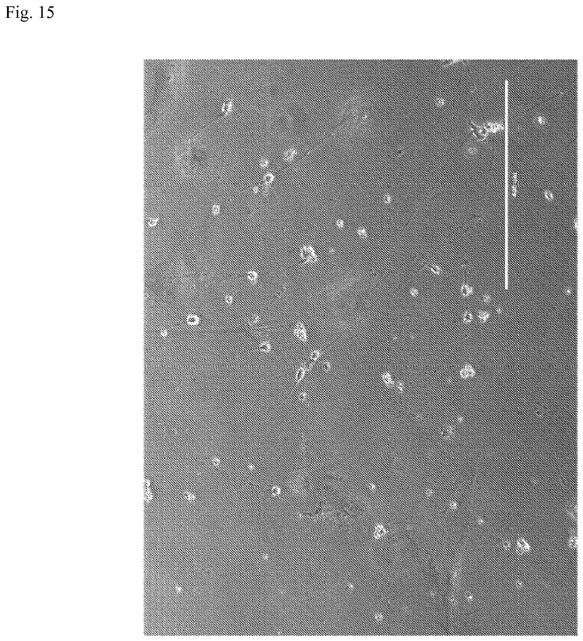

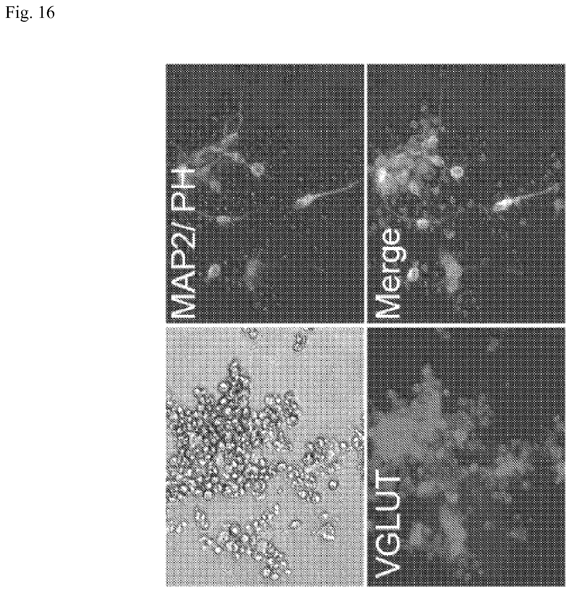

Induction of neurons was confirmed as a result, as shown in Fig. 15. When the cells were immunostained in the same manner as Example 1 of the second embodiment, the induced neurons were confirmed to be expressing the neuron marker MAP2 and the excitatory neuron marker vGLUT, as shown in Fig. 16.

## Claims

1. A nerve cell production method that includes:
preparing stem cells, and
introducing inducing factor RNA into the stem cells and causing their differentiation into nerve cells.

2. The nerve cell production method according to claim 1, wherein the stem cells are induced pluripotent stem cells.

3. The nerve cell production method according to claim 1 or 2, wherein the nerve cells are neurons.

4. The nerve cell production method according to claim 1 or 2, wherein the nerve cells are inhibitory neurons.

5. The nerve cell production method according to claim 1 or 2, wherein the nerve cells are excitatory neurons.

6. The nerve cell production method according to claim 1 or 2, wherein the nerve cells are dopamine-producing neurons.

7. The nerve cell production method according to any one of claims 1 to 6, wherein the inducing factor RNA is introduced into the stem cells by a lipofection method.

8. The nerve cell production method according to any one of claims 1 to 7, wherein the inducing factor RNA includes mRNA corresponding to a drug resistance gene.

9. The nerve cell production method according to claim 8, wherein, after the inducing factor RNA has been introduced into the stem cells, cells that exhibit resistance to the drug are selected.

10. A nerve cell production method including:
preparing cells,
introducing reprogramming factor RNA into the cells, and
introducing inducing factor RNA into the cells in which the reprogramming factor RNA has been introduced, to cause their differentiation into nerve cells.

11. The nerve cell production method according to claim 10, wherein the reprogramming factor RNA is introduced into the cells and the inducing factor RNA is introduced into the reprogramming factor RNA-introduced cells, all in the same culturing vessel.

12. The nerve cell production method according to claim 11, wherein, after the reprogramming factor RNA has been introduced into the cells, the inducing factor RNA is introduced into the reprogramming factor RNA-introduced cells without detaching the reprogramming factor RNA-introduced cells from the culturing vessel.

13. The nerve cell production method according to claim 11, wherein, after the reprogramming factor RNA has been introduced into the cells, the inducing factor RNA is introduced into the reprogramming factor RNA-introduced cells after detaching the reprogramming factor RNA-introduced cells from the culturing vessel and seeding the reprogramming factor RNA-introduced cells into a different culturing vessel.

14. The nerve cell production method according to any one of claims 10 to 13, wherein the cells are human fibroblasts.

15. The nerve cell production method according to any one of claims 10 to 13, wherein the cells are blood cells.

16. The nerve cell production method according to any one of claims 10 to 15, wherein the nerve cells are inhibitory neurons.

17. The nerve cell production method according to any one of claims 10 to 15, wherein the nerve cells are excitatory neurons.

18. The nerve cell production method according to any one of claims 10 to 15, wherein the nerve cells are dopamine-producing neurons.

19. The nerve cell production method according to any one of claims 10 to 18, wherein the inducing factor RNA is introduced into the cells by a lipofection method.

20. The nerve cell production method according to any one of claims 10 to 19, wherein the inducing factor RNA includes mRNA corresponding to a drug resistance gene.

21. The nerve cell production method according to claim 20, wherein, after the inducing factor RNA has been introduced into the stem cells, cells that exhibit resistance to the drug are selected.

22. RNA corresponding to DNA of any one of SEQ ID NO: 1 to 10.

23. An inducing factor comprising RNA corresponding to DNA of any one of SEQ ID NO: 1 to 10.
